# EUROPEAN PATENT SPECIFICATION

(11) **EP 3 130 682 B1**
(45) Date of publication and mention of the grant of the patent: **18.03.2020**
(21) Application number: 16184101.0
(22) Date of filing: 12.08.2016
(51) Int. Cl.: C12Q 1/6834

(54) **METHODS FOR GENERATING POLYMER ARRAYS**
VERFAHREN ZUR ERZEUGUNG VON POLYMER-ARRAYS
PROCÉDÉS DE GÉNÉRATION DE MATRICES POLYMÈRES

(30) Priority: 13.08.2015 US 201562204937 P; 12.11.2015 US 201562254589 P
(43) Date of publication of application: 15.02.2017
(73) Proprietor: Centrillion Technology Holdings Corporation, Grand Cayman, KY1-1104 (KY)
(72) Inventor: POLLOM, Thomas Scott, Menlo Park, CA 94025 (US); ZHOU, Wei, Saratoga, CA 95070 (US)
(74) Representative: J A Kemp LLP

(56) References cited:
- EP-A1- 1 964 947
- US-A- 5 143 854
- US-A1- 2002 102 564
- US-A1- 2008 193 863
- US-B1- 6 610 482
- S. SRINIVASAN ET AL: "A Review of Algorithms for Border Length Minimization Problem", IETE TECHNICAL REVIEW., vol. 31, no. 5, 3 September 2014 (2014-09-03), pages 369-382, XP055327421, ISSN: 0256-4602, DOI: 10.1080/02564602.2014.959078
- FELDMAN W ET AL: "Gray Code Masks for Sequencing by Hybridization", GENOMICS, ACADEMIC PRESS, SAN DIEGO, US, vol. 23, no. 1, 1 September 1994 (1994-09-01), pages 233-235, XP024796813, ISSN: 0888-7543, DOI: 10.1006/GENO.1994.1482 [retrieved on 1994-09-01]
- HANNENHALLI S ET AL: "COMBINATORIAL ALGORITHMS FOR DESING OF DNA ARRAYS", ADVANCES IN BIOCHEMICAL ENGINEERING, BIOTECHNOLOGY, SPRINGER, BERLIN, DE, vol. 77, 1 January 2002 (2002-01-01), pages 1-19, XP008011063, ISSN: 0724-6145
- VAMSI KUNDETI ET AL: "Border Length Minimization Problem on a Square Array", JOURNAL OF COMPUTATIONAL BIOLOGY., vol. 21, no. 6, 1 June 2014 (2014-06-01), pages 446-455, XP055327433, US ISSN: 1066-5277, DOI: 10.1089/cmb.2013.0127
- A. B. Kahng ET AL: "Border Length Minimization in DNA Array Design*" In: "Network and Parallel Computing", 1 January 2002 (2002-01-01), Springer International Publishing, Cham 032548, XP055327437, ISSN: 0302-9743 ISBN: 978-3-642-34690-3 vol. 2452, pages 435-448, DOI: 10.1007/3-540-45784-4_34, * the whole document *

## Description

### BACKGROUND

Large array of polymeric molecules have wide ranging applications and are of substantial importance to the medical, biotechnology and pharmaceutical industries. For example, arrays of oligonucleotide probes are proving to be a powerful tool for large-scale DNA and RNA sequence analysis. The field of nucleic acid assays has been transformed by microarrays which allow monitoring of gene expression events, expression profiling, diagnostic and genotyping analyses, among other applications. Substrates bearing arrays of nucleic acid probes need to be manufactured in a manner that allows assays such as expression monitoring, genotyping and other studies to be performed accurately and efficiently. With more sensitive applications being contemplated for microarrays in the fields of pharmacogenomics and diagnostics, for example, there exists a need in the art for methods and technologies for producing polymeric arrays with increased accuracy, efficiency and lower cost.

### SUMMARY

In general, when designing microarrays, the designer is confronted with the "Border Length Minimization Problem" (BLMP), for example, how to place a given N x N strings of the same length in a grid of size N x N such that the Hamming distance summed over all the pairs of neighbors in the grid can be minimized. (Kundeti et al., "Border Length Minimization Problem on a Square Array," Journal of Computational Biology 21.6 (2014): 446-455) As discussed in the paper, reducing this sum of Hamming distances between adjacent embeddings can reduce the number of synthesis errors.

Feldman *et al.* produced a mask set trying to minimize the "Border Length" for a chip. (Feldman et al., "Gray code masks for sequencing by hybridization." Genomics 23.1 (1994): 233-235) However, the fact that the resulting chip contains all possible n-mers makes it unsuitable for use, e.g., as a source of oligonucleotide barcodes with given constraints.

Recognized herein is the need for fabricating a chip containing polymer array[s] in which the "Border Length" can be minimized and the resulting polymers are useful in applications such as oligonucleotide barcodes. This minimization of "Border Length" can be especially important for applications in which chips having no spaces between features are required. The present disclosure provides methods for generating polymers (such as DNA sequences) with error-correcting capabilities. The methods may comprise creating mask sets in which edit distances (e.g., Hamming distance) of adjacent embeddings can be all equal to 1 (1 is the minimum possible edit distance between pairs of embeddings in cases where all of the embeddings are unique), so that the sum of the edit distances can be at its absolute minimum, and, equivalently, the "Border Length" can be at its absolute minimum. The methods of the present disclosure can also reduce risk of errors during polymer (such as DNA sequences) synthesis.

An aspect of the present disclosure provides an array comprising at least 1,000 different polymers, each coupled to a distinct location on a surface, wherein each polymer differs from polymers adjacent to it by at most 5 subunits. In some embodiments of aspects provided herein, each polymer differs from polymers adjacent to it by one and only one subunit. In some embodiments of aspects provided herein, a first polymer differs from an adjacent second polymer by insertions, deletions, substitutions, and/or translocation of single subunits. In some embodiments of aspects provided herein, the array comprises at least 10,000 polymers. In some embodiments of aspects provided herein, the array comprises at least 100,000 polymers. In some embodiments of aspects provided herein, each of the polymers comprises at least 10 subunits. In some embodiments of aspects provided herein, each of the polymers comprises at least 20 subunits. In some embodiments of aspects provided herein, each of the polymers comprises at least 50 subunits. In some embodiments of aspects provided herein, each of the polymers is adjacent to at least two other polymers. In some embodiments of aspects provided herein, each of the polymers is adjacent to at least three other polymers. In some embodiments of aspects provided herein, polymers immobilized at two nonadjacent locations differ from each other by at least the same number of insertions, deletions, substitutions, and/or translocations of single subunits as the number of locations between the two nonadjacent locations. In some embodiments of aspects provided herein, polymers immobilized at two nonadjacent locations have a differing number of subunits that is at least the same number as the number of locations between the two locations. In some embodiments of aspects provided herein, the polymers are arranged on the surface in a two-dimensional pattern with n rows and m columns, wherein n and m are integers. In some embodiments of aspects provided herein, n is at least 30. In some embodiments of aspects provided herein, n is at least 1,000. In some embodiments of aspects provided herein, n is at least 5,000. In some embodiments of aspects provided herein, m is at least 30. In some embodiments of aspects provided herein, m is at least 1,000. In some embodiments of aspects provided herein, m is at least 5,000. In some embodiments of aspects provided herein, each of the polymers comprises a first segment, a second segment and a third segment between the first segment and the second segment, each of the segments comprising at least two subunits. In some embodiments of aspects provided herein, the first segment is adjacent to the surface and the second segment is distal the surface. In some embodiments of aspects provided herein, each of the polymers has the same third segment. In some embodiments of aspects provided herein, polymers immobilized at adjacent locations in the same column have the same first segment and differ in the second segment by at most 5 subunits. In some embodiments of aspects provided herein, the polymers differ in the second segment by at most 5 insertions, deletions, substitutions, and/or translocations of single subunits. In some embodiments of aspects provided herein, the polymers differ in the second segment by one and only one subunit. In some embodiments of aspects provided herein, the polymers differ in the second segment by an insertion, deletion, substitution, or translocation of a single subunit. In some embodiments of aspects provided herein, polymers immobilized at adjacent locations in the same row have the same second segment and differ in the first segment by at most 5 subunits. In some embodiments of aspects provided herein, the polymers differ in the first segment by at most 5 insertions, deletions, substitutions, and/or translocations of single subunits. In some embodiments of aspects provided herein, the polymers differ in the first segment by one and only one subunit. In some embodiments of aspects provided herein, the polymers differ in the first segment by an insertion, deletion, substitution, or translocation of a single subunit. In some embodiments of aspects provided herein, polymers immobilized at two nonadjacent locations in the same column have the same first segment and differ from each other in the second segment by at least the same number of insertions, deletions, substitutions, and/or translocations of single subunits as the number of locations between the two nonadjacent locations. In some embodiments of aspects provided herein, polymers immobilized at two nonadjacent locations in the same column differ in the number of subunits of the second segment by at least the same number as the number of locations between the two nonadjacent locations. In some embodiments of aspects provided herein, polymers immobilized at two nonadjacent locations in the same row have the same second segment and differ from each other in the first segment by at least the same number of insertions, deletions, substitutions, and/or translocations of single subunits as the number of locations between the two nonadjacent locations. In some embodiments of aspects provided herein, polymers immobilized at two nonadjacent locations in the same row differ in the number of subunits of the first segment by at least the same number as the number of locations between the two locations. In some embodiments of aspects provided herein, each of the polymers is located in an area of less than 100 µm². In some embodiments of aspects provided herein, each of the polymers is located in an area of less than 10 µm². In some embodiments of aspects provided herein, each of the polymers is located in an area of less than 5 µm². In some embodiments of aspects provided herein, the polymers are arranged in square configurations. In some embodiments of aspects provided herein, the polymers are arranged in rectangular configurations. In some embodiments of aspects provided herein, at least 50% of the polymers are located in distinct locations that have the same size. In some embodiments of aspects provided herein, the polymers comprise nucleic acid molecules. In some embodiments of aspects provided herein, the polymers are selected from the group consisting of DNA, RNA, PNA, LNA, and a hybrid thereof. In some embodiments of aspects provided herein, the polymers are single-stranded or double-stranded.

Another aspect of the present disclosure provides a method for synthesizing an array of at least 1,000 polymers each coupled to a distinct location on a substrate, comprising: (a) providing a substrate having a plurality of distinct locations; (b) providing a set of masks, each mask of the set defining a different subset of the plurality of distinct locations on the substrate; (c) using a computer executable logic selecting a mask from the set of masks to overlay the substrate; (d) using the computer executable logic selecting one or more subunits to be introduced onto the substrate at a defined subset of the plurality of distinct locations using the selected mask; (e) performing polymer synthesis on the substrate at the defined subset of the plurality of distinct locations using the one or more subunits; and (f) repeating steps (b)-(e) at least 10 times, thereby generating an array of at least 1,000 polymers, each couple to one of the plurality of distinct locations.

In some embodiments of aspects provided herein, the array comprises at least 10,000 polymers. In some embodiments of aspects provided herein, each of the plurality of distinct locations has an area of less than 5 µm². In some embodiments of aspects provided herein, at least 90% of the plurality of distinct locations has the same area. In some embodiments of aspects provided herein, each of the plurality of distinct locations has the same area. In some embodiments of aspects provided herein, each of the plurality of distinct locations is adjacent to at least two other distinct locations. In some embodiments of aspects provided herein, each individual mask of the set comprises a plurality of openings which define a pattern of active and inactive regions on the substrate, and the one or more subunits are only added to the active regions of the substrate during synthesis. In some embodiments of aspects provided herein, the each individual mask covers all the distinct locations on the substrate. In some embodiments of aspects provided herein, the openings are aligned in a single direction. In some embodiments of aspects provided herein, each of the openings covers an integer number of the distinct locations and has the same shape. In some embodiments of aspects provided herein, each of the openings has a rectangular shape. In some embodiments of aspects provided herein, each of the openings has a width of at least 0.5 µm. In some embodiments of aspects provided herein, at least 20% of the openings have differing widths. In some embodiments of aspects provided herein, at least 50% of the openings have differing widths. In some embodiments of aspects provided herein, each of the openings has a length of at least 500 µm. In some embodiments of aspects provided herein, at least 50% of the openings have the same length. In some embodiments of aspects provided herein, at least 90% of the openings have the same length. In some embodiments of aspects provided herein, a first polymer differs from an adjacent second polymer by at most 5 insertions, deletions, substitutions, and/or translocations of single subunits. In some embodiments of aspects provided herein, the first polymer differs from the adjacent second polymer by one and only one insertion, deletion, substitution or translocation of a single subunit. In some embodiments of aspects provided herein, each of the polymers is formed with a unique string of synthetic steps defined by the set of masks, and two strings of synthetic steps used to form neighboring polymers in adjacent locations differ from each other by at most 5 synthetic steps. In some embodiments of aspects provided herein, the two strings of synthetic steps differ from each other by one and only one synthetic step. In some embodiments of aspects provided herein, the method further comprises, before step (b), providing a computer readable medium comprising codes that, upon execution by one or more computer processors, implement a method for generating mask design files, the mask design files defining a pattern of openings on each individual mask of the set. In some embodiments of aspects provided herein, the method further comprises converting the mask design files into physical masks. In some embodiments of aspects provided herein, each of the polymers of the array comprises a first segment, a second segment, and a common third segment between the first segment and the second segment, each of the segments comprising at least two subunits. In some embodiments of aspects provided herein, the same set of masks is used for forming both the first segment and the second segment of the polymers. In some embodiments of aspects provided herein, a first set and a second set of masks are provided for forming the first segment and the second segment of the polymers respectively, and the openings comprised in the first set and the second set of masks are aligned in two directions orthogonal to each other. In some embodiments of aspects provided herein, the method further comprises providing a separate mask designed to expose all of the distinct locations on the substrate for forming the third segment of the polymers. In some embodiments of aspects provided herein, the substrate comprises a material selected from the group consisting of silicon nitrides, silica and glass. In some embodiments of aspects provided herein, the substrate is part of a chip. In some embodiments of aspects provided herein, each mask of the set comprises a material selected from the group consisting of polymeric, semiconductor and metallic materials. In some embodiments of aspects provided herein, each mask of the set has a thickness in the range of 50 µm -100 mm. In some embodiments of aspects provided herein, (e) further comprises (i) providing a light source and positioning the selected mask along an optical path between the light source and the substrate, thereby defining a pattern of active regions and inactive regions on the substrate during a single step of the polymer synthesis; and (ii) directing a light beam from the light source to the substrate to perform light-directed synthesis in the locations within the active regions on the substrate. In some embodiments of aspects provided herein, the light source is in the range of ultraviolet to near ultraviolet wavelengths. In some embodiments of aspects provided herein, each of the polymers comprises at least 15 subunits. In some embodiments of aspects provided herein, each of the polymers comprises at least 20 subunits.

Additional aspects and advantages of the present disclosure will become readily apparent to those skilled in this art from the following detailed description, wherein only illustrative embodiments of the present disclosure are shown and described. As will be realized, the present disclosure is capable of other and different embodiments, and its several details are capable of modifications in various obvious respects, all without departing from the disclosure. Accordingly, the drawings and description are to be regarded as illustrative in nature, and not as restrictive.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:
**FIG. 1A** shows an example polymer comprising a upper segment and a lower segment, separated by a middle segment;
**FIG. 1B** shows an example substrate having an array of distinct locations of the present disclosure;
**FIG. 2** shows an example mask of the present disclosure;
**FIG. 3A** illustrates an example multi-step fabrication method of the present disclosure (Figure discloses SEQ ID NOS 5, 6, and 6, respectively, in order of appearance);
**FIG. 3B** shows example embeddings and synthesized polymers using the example embeddings (Figure discloses SEQ ID NOS 7-10, respectively, in order of appearance);
**FIG. 4** shows an example workflow for generating mask design files;
**FIG. 5** schematically illustrates a computer system that is programmed or otherwise configured to implement systems and methods of the present disclosure;
**FIGs. 6A-6D** show an example procedure to synthesize polymers (Figure 6A discloses SEQ ID NO: 11);
**FIG. 7** illustrates an example polymeric barcode synthesized by using a set of masks (Figure discloses SEQ ID NOS 12-13, respectively, in order of appearance);
**FIG. 8** shows example embeddings and resulting DNA sequences generated using the methods of the present disclosure;
**FIG. 9** shows an example embedding generating method and resulting DNA sequences (Figure discloses SEQ ID NOS 14-17, respectively, in order of appearance);
**FIG. 10** shows an example embedding generating method and resulting DNA sequences having multiple segments (Figure discloses SEQ ID NOS 18-21, respectively, in order of appearance); and
**FIG. 11** illustrates an example method for generating polymers having multiple segments using concatenated embeddings.

### DETAILED DESCRIPTION

While various embodiments of the invention have been shown and described herein, it will be obvious to those skilled in the art that such embodiments are provided by way of example only. Numerous variations, changes, and substitutions may occur to those skilled in the art. It should be understood that various alternatives to the embodiments of the disclosure described herein may be employed.

### Definitions

As used herein, the singular form "a", "an", and "the" include plural references unless the context clearly dictates otherwise.

As used herein, the term "about" refers to the indicated numerical value ±10%.

As used herein, open terms, for example, "comprise", "contain", "include", "including", "have", "having" and the like refer to comprising unless otherwise indicates.

As used herein, the term "embedding" and "a string of synthetic steps" refer to a series of active and inactive steps designed for forming an individual polymer on the substrate and can be used interchangeably. For example, in cases where light-directed synthetic methods are employed, the "embedding" refer to a series exposure and non-exposure steps.

As used herein, the term "edit distance" refers to the minimum number of changes (such as insertions, deletions, substitutions and translocations) needed to convert one polymer into another. For example, the edit distance between sequences AGCGCTTAGCCTAGAGCTCTAG (SEQ ID NO: 1) and GCGCTTAGCTTAGAGCTCTATTG (SEQ ID NO: 2) is 4.

As used herein, the term "polymer" refers to any kind of natural or non-natural large molecules, composed of multiple subunits. Polymers may comprise homopolymers, which contain only a single type of repeating subunits, and copolymers, which contain a mixture of repeating subunits. In some cases, polymers are biological polymers that are composed of a variety of different but structurally related subunits, for example, polynucleotides such as DNA composed of a plurality of nucleotide subunits.

As used herein, the term "subunit" refers to a subdivision of a larger molecule or a single molecule that assembles (or "coassembles") with other molecules to form a larger molecular complex such as polymers. Non-limiting example of subunits include monomers, simple carbohydrates or monosaccharide moieties, fatty acids, amino Acids, and nucleotides.

As used herein, the term "nucleic acid" generally refers to a polymer comprising one or more nucleic acid subunits or nucleotides. A nucleic acid may include one or more subunits selected from adenosine (A), cytosine (C), guanine (G), thymine (T) and uracil (U), or variants thereof. A nucleotide can include A, C, G, T or U, or variants thereof. A nucleotide can include any subunit that can be incorporated into a growing nucleic acid strand. Such subunit can be an A, C, G, T, or U, or any other subunit that is specific to one or more complementary A, C, G, T or U, or complementary to a purine (i.e., A or G, or variant thereof) or a pyrimidine (i.e., C, T or U, or variant thereof). A subunit can enable individual nucleic acid bases or groups of bases (e.g., AA, TA, AT, GC, CG, CT, TC, GT, TG, AC, CA, or uracil-counterparts thereof) to be resolved. In some examples, a nucleic acid is deoxyribonucleic acid (DNA) or ribonucleic acid (RNA), or derivatives thereof. A nucleic acid may be single-stranded or double-stranded.

As used herein, the term "adjacent" or "adjacent to," includes 'next to', 'adjoining', and "abutting". In one example, a first location is adjacent to a second location when the first location is in direct contact and shares a common border with the second location and there is no space between the two locations. In some cases, the adjacent is not diagonally adjacent.

### Polymer Array

An aspect of present disclosure provides an array of polymers which can be used for performing multiplex assay. The array may comprise at least 100, 250, 500, 750, 1,000, 2,000, 3,000, 4,000, 5,000, 6,000, 7,000, 8,000, 9,000, 10,000, 20,000, 30,000, 40,000, 50,000, 60,000, 70,000, 80,000, 90,000, 100,000, 120,000, 140,000, 160,000, 180,000, 200,000, 250,000, 300,000, 350,000, 400,000, 450,000, 500,000, 600,000, 700,000, 800,000, 900,000, 1,000,000, 10,000,000, 20,000,000, 30,000,000, 40,000,000, 50,000,000, 60,000,000, 70,000,000, 80,000,000, 90,000,000, 100,000,000, 200,000,000, 300,000,000, 400,000,000, 500,000,000, 600,000,000, 700,000,000, 800,000,000, 900,000,000, 1,000,000,000, 2,000,000,000, 3,000,000,000, 4,000,000,000, 5,000,000,000, or more unique polymeric molecules. In some cases, the number of unique polymeric molecules in the array may be between any of the two values described herein, for example, about 150,000 or 250,000,000.

Each polymer of the array may be immobilized at a distinct location on a substrate. Each of the distinct locations on the substrate may be adjacent to at least one other distinct location. In some cases, each distinct location may be adjacent to at least two, three, four, five, or more other distinct locations. In some cases, a certain percentage (e.g., at least 10%, 20%, 30%, 40%, 50%, 55%, 60%, 65%, 70%, 75%, 80%, 85%, 90%, 95%, or more) of the distinct locations on the subject may be adjacent to at least one, two, three, four or more other distinct locations.

Polymers immobilized at distinct locations may be different, and each of the polymers may differ from adjacent polymers by a maximum number of single subunits. For example, in some cases, each polymer differs from adjacent polymers (i.e., polymers immobilized on/couple with adjacent locations) by at most 500, 400, 300, 200, 100, 90, 80, 70, 60, 50, 45, 40, 35, 30, 25, 20, 18, 16, 14, 12, 10, 9, 8, 7, 6, 5, 4, 3, or 2 subunits, including substitutions, insertions, deletions, and/or translocations of single subunits. In some cases, each of the polymers differs from its adjacent polymers by one and only one subunit. By "adjacent polymers" we mean polymers immobilized at adjacent locations of a given location on the substrate. In some cases, a first polymer may differ from a second polymer immobilized at an adjacent location by a substitution, insertion, deletion, or translocation of a single subunit.

Each polymer of the array may comprise more than 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 45, 50, 55, 60, 65, 70, 75, 80, 85, 90, 95, 100, 120, 140, 160, 180, 200, 300, 400, 500, 600, 700, 800, 900, or 1,000 subunits. The subunits may or may not be identical.

Polymers of the array may have the same or varying length(s) (i.e., having the same or different numbers of subunit(s)). For example, in some cases, greater than or equal to 10%, 20%, 30%, 40%, 50%, 60%, 70%, 80%, 90%, or more of the polymers have the same or different length(s). In some cases, less than or equal to about 100%, 95%, 90%, 80%, 70%, 60%, 50%, 40%, 30%, 20%, 10% or less of the polymers have the same or different length(s). In some cases, the percentage of polymers that have the same or different length(s) may be between any of the two values provided herein, for example, about 55%, 65%, or 75%.

Each polymer of the array may comprise more than one segment, each of which may comprise one or more subunits. For example, each polymer may comprise a first segment and a second segment, separated by a third segment. In some cases, some or all of the polymers of the array share a common third segment with known sequences of subunits. The polymers may be immobilized at an array of distinct locations arranged in a pattern, e.g., a pattern with rows and columns. The polymers immobilized at adjacent locations in the same column may have the same first/second segment, and differ in the second/first segments by a maximum number of subunits, for example, by at most 500, 400, 300, 200, 100, 90, 80, 70, 60, 50, 45, 40, 35, 30, 25, 20, 18, 16, 14, 12, 10, 9, 8, 7, 6, 5, 4, 3, or 2 subunits, including substitutions, insertions, deletions, and/or translocations of single subunits. In some examples, the polymers immobilized at adjacent locations in the same column have the same first/second segment, and differ in their second/first segments by one and only one subunit (including e.g., a substitution, insertion, deletion, or translocations of a single subunit). Similarly, in some cases, the polymers immobilized at adjacent locations in the same row may have the same second/first segment, and differ in the first/second segments by a maximum number of subunits. In some examples, the polymers immobilized at adjacent locations in the same row have the same second/first segment, and differ in their first/second segments by one and only one subunit.

Additionally, the polymers immobilized at two nonadjacent locations in the same column may have the same first/second segment, and differ in the number of subunits of the second/first segment by at least the same number as the number of distinct locations between the two nonadjacent locations. The polymers immobilized at two nonadjacent locations in the same row may have the same second/first segment, and differ in the number of subunits of the first/second segment by at least the same number as the number of distinct locations between the two nonadjacent locations. For example, polymers immobilized at two distinct locations in the same column which have 6 other distinct locations in between, may have the same first/second segment, while differ in the number of subunits of the second/first segment by at least 6 subunits. The subunit difference may include substitutions, insertions, deletions, and/or translocations of single subunits.

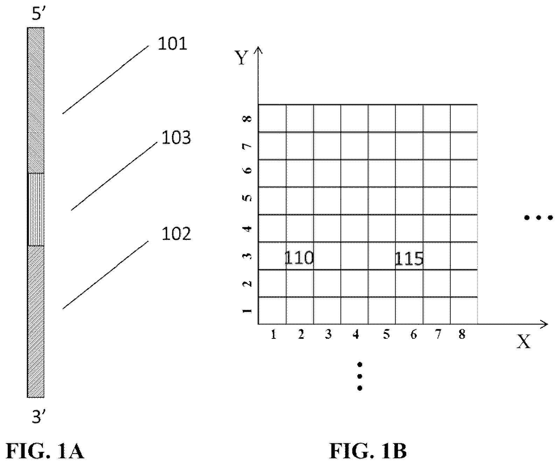

FIG. 1A shows an example polymer of the present disclosure. In FIG. 1A, a polymer comprises a first segment 101 and a second segment 102, which are separated by a third segment 103. The first segment 101, second segment 102 and third segment 103 can be of any length and incorporate therein any type/number of monomers or subunits. For example, a polymer molecule can be a nucleic acid molecule which includes a first segment (or an upper segment) GCAGTGCCACAGA (SEQ ID NO: 3) and a second segment (or a lower segment) CAACAACTGA (SEQ ID NO: 4), separated by a third segment with a known sequence TTT. In some cases, the sole purpose of a known sequence (e.g., sequence 103) is to distinguish between the two segments of the polymeric molecules (e.g., upper and lower segments 101 and 102 in FIG. 1A). To avoid confusion, the sequence used to distinguish the two segments is designed such that neither of the two segments contains the same such sequence. As described above and elsewhere herein, differences between sequences of polymers immobilized at two distinct locations (either adjacent or nonadjacent) can be determined by the relative position of the two locations. In cases where more than one segments are comprised in polymeric molecules, differences between each segment of the polymers immobilized at two distinct locations can also be determined, for example, by the number of distinct locations between the two locations. In cases where a coordinate system is used to determine the position for each distinct location, each distinct location may be assigned a unique coordinate and such coordinate may be used to determine a difference between polymer sequences. Each coordinate may further comprise one or more sub-coordinates. For example, in cases where the locations are arranged in an array with rows and columns, each unique coordinate may further comprise a horizontal coordinate, and a vertical coordinate, both of which may be integers. The horizontal and vertical coordinate may be used to calculate a difference in the number of subunits of the first and second segment of polymers immobilized at two distinct locations, respectively.

FIG. 1B shows an example array of locations. As illustrated in the figure, a plurality of distinct locations is arranged as a square lattice, wherein each unit cell has the same side length. The side length of the unit cell can vary, spanning from 1 nm to a few millimeters. For example, the side length of the unit cell may be greater than or equal to about 1 nm, 5 nm, 10 nm, 20 nm, 40 nm, 60 nm, 80 nm, 100 nm, 200 nm, 400 nm, 600 nm, 800 nm, 1 µm, 2 µm, 3 µm, 4 µm, 5 µm, 6 µm, 7 µm, 8 µm, 9 µm, 10 µm, 15 µm, 20 µm, 25 µm, 30 µm, 35 µm, 40 µm, 45 µm, 50 µm, 60 µm, 70 µm, 80 µm, 90 µm, 100 µm, 200 µm, 300 µm, 400 µm, 500 µm, 600 µm, 700 µm, 800 µm, 900 µm, 1 mm, 5 mm, 10 mm, or more. In some cases, the side length of the unit cell may be smaller than or equal to about 50 mm, 25 mm, 10 mm, 5 mm, 1 mm, 800 µm, 600 µm, 400 µm, 200 µm, 100 µm, 75 µm, 50 µm, 40 µm, 30 µm, 20 µm, 10 µm, 8 µm, 6 µm, 4 µm, 2 µm, 1 µm, 750 nm, 500 nm, 250 nm, 100 nm, 75 nm, 50 nm, 25 nm, 10 nm, 5 nm, 1 nm, or less. In some cases, the side length of the unit cell may be in between any of the two values described herein, for example, about 1.5 µm.

A coordinate system is employed to determine a unique coordinate for each of the locations. Each of the locations may comprise one or more polymers and the polymers immobilized at the same location have the same coordinate. In this example, each polymer has two segments (e.g., an upper segment and a low segment as shown in FIG. 1A), and each location has its unique coordinate which further comprises an X-coordinate and a Y-coordinate. The X- and Y-coordinates are used to determine the minimum edit distance between (including e.g., differences in the number of subunits) the lower and upper segments of the polymers immobilized at two distinct locations, respectively. For example, the differences between the upper segments and lower segments of two polymeric molecules immobilized at locations with coordinates (x1, y1) and (x2, y2) can be determined in the following way: (i) if |x1-x2| ≤ 4, then the polymers differ in the lower segments by |x1-x2| of subunits; (ii) if |x1-x2| > 4, then the polymers differ in the number of subunits of lower segment by at least 4; (iii) if |y1-y2| ≤ 4, then the polymers differ in the upper segments by |y1-y2| of subunits; or (iv) if |y1-y2| > 4, then the polymers differ in the number of subunits of upper segment by at least 4.

In FIG. 1B, the coordinates of locations 110 and 115 are (2, 3) and (6, 3), respectively. Therefore, with the coordinates, it can be determined that the polymeric molecules in these two locations (i.e., 110 and 115) have the same upper segment but different lower segments with an edit distance of at least 4.

As provided herein, some or all of the distinct locations may comprise one or more polymers and the polymer immobilized at the same location may be identical. For example, in some cases, at least 1%, 5%, 10%, 20%, 40%, 50%, 60%, 70%, 80%, 90%, 99% or more of the distinct locations comprise 1, 2, 3, 4, 5, 6, 7, 8, 9, 10, 11, 12, 13, 14, 15, 16, 17, 18, 19, 20, 30, 40, 50, 60, 70, 80, 90, 100, 200, 300, 400, 500, 600, 700, 800, 1000 polymers, or more. In cases where more than one polymer is immobilized at a distinct location, the polymers are identical except for an error caused by e.g., inefficiencies during polymer synthesis. An error rate, defined as a ratio of the total number of polymers with errors to that of polymers with correct sequences, may be used for screening the polymer arrays before use. For each polymer array, prior to its application, an error rate may be determined and compared with a pre-determined threshold (e.g., 5%, 3%, 1%, 0.5%, 0.1%, 0.01%, or 0.001%), and only the array that has the error rate below the pre-determined threshold can be released for further uses. Differences between polymer sequences immobilized at two nonadjacent locations may be determined by a relative position of the two locations. In some cases, the polymers immobilized at two nonadjacent locations may differ in polymer sequences by at least the same number of locations between the two nonadjacent locations. For example, polymers immunized at two locations having at least 5 other distinct locations in between may differ in the sequences by at least 5 subunits, including substitutions, insertions, deletions, and/or translocations of the subunits.

In some cases, relative positions of two locations can be determined by calculating a difference between the two positions which can be measured or identified by a position locator. The position locator may comprise a coordinate system which uses one or more numbers, or coordinates, to determine a unique position for each distinct location. In some cases, each location can be mapped 1-to-1 to the polymer sequence it contains, so that e.g. if the sequence of a polymer is determined, the distinct location at which the polymer immobilized is known.

The locations can take various shapes, such as round, square, rectangle, polygon, elliptical, elongated bar, polygon, or any other regular or irregular shapes or combinations thereof. Area of each individual location or site may vary. In some cases, each of the locations has an area of greater than or equal to about 1 nanometer (nm)², 10 nm², 100 nm², 500 nm², 1000 nm², 10,000 nm², 50,000 nm², 1 micron (µm)², 5 µm², 10 µm² 20 µm² 30 µm² 40 µm² 50 µm² 60 µm² 70 µm² 80 µm² 90 µm², 100 µm², 200 µm², 300 µm², 400 µm², 500 µm², 600 µm², 700 µm², 800 µm², 900 µm², 1,000 µm², 2,000 µm², 4,000 µm², 6,000 µm², 8,000 µm², 10,000 µm², 25,000 µm², 50,000 µm², 75,000 µm², 100,000 µm², or more. In some cases, each of the locations has an area of smaller than or equal to about 1,000,000 µm², 500,000 µm², 100,000 µm², 50,000 µm², 10,000 µm², 7,500 µm², 5,000 µm², 2,500 µm², 1,000 µm², 750 µm², 500 µm², 250 µm², 100 µm², 80 µm², 60 µm², 40 µm², 20 µm², 10 µm², 5 µm², 1 µm², 75,000 nm², 50,000 nm², 25,000 nm², 10,000 nm², 5,000 nm², 1,000 nm², or less. In some cases, each individual location may have an area in between any of the two values described herein.

The distinct locations may be arranged in an array on the substrate. The array may be in any pattern, such as a linear pattern, a two-dimensional pattern (e.g., oblique, rectangular, centered rectangular, hexagonal (rhombic), and square lattice, or a pattern with n rows and m columns), or any regular or irregular patterns. In cases where the locations are arranged in a pattern of n rows and m columns, any number of rows and columns may be used. In some cases, n and/or m is greater than or equal to 1, 5, 10, 20, 30, 40, 50, 60, 70, 80, 90, 100, 200, 300, 400, 500, 600, 700, 800, 900, 1,000, 2,000, 3,000, 4,000, 5,000, 6,000, 7,000, 8,000, 9,000, 10,000, 20,000, 30,000, 40,000, 50,000, 60,000, 70,000, 80,000, 90,000, 100,000 or more. In some cases, n and/or m is smaller than or equal to 1,000,000, 500,000, 250,000, 100,000, 75,000, 50,000, 25,000, 10,000, 7,500, 5,000, 2,500, 1,000, 750, 500, 250, 100, 80, 60, 40, 20, 10, 5, or less. In some cases, n and/or m can be any number in between any of the two values described above, e.g., 15, 150, or 3,500.

The substrate may be solid or semi-solid. The substrate may comprise one or more layers made of the same or different materials, such as metals, glass, semiconductors, synthetic or natural materials, and organic or inorganic materials. Non-limiting examples of materials that can be used to form the substrate may comprise glass, quartz, silicon, a silicon-based material (e.g., silicon nitride or silica), a metal, plastics, polymeric materials (e.g., thermoset, elastomer, thermoplastic, polystyrene, nylon, polydopamine (PDA), polyvinyl chloride (PVC), poly(dimethylsiloxane) (PDMS), polyvinylidene fluoride etc.), paper, hydrogel, or a combination thereof. The substrate can take various shapes, 1-, 2-, or 3-dimensional, such as sheet, sphere, cube, cuboid, cone, cylinder, prism, pyramid, tube, plate, disc, rod, or any regular or irregular shapes. In some cases, the substrate is part of a chip. The chip may comprise millions of micron-scale features, each of which contains thousands of copies of a unique polymer, i.e., a DNA sequence.

The substrate may further comprise a surface. The surface of the substrate may be a flat surface, a curve surface, or a surface with raised and/or depressed regions which may facilitate the implementation of the methods of the present disclosure. The raised/depressed regions on the surface can be continuous, semi-continuous, or discontinuous. In some cases, the surface of the substrate may have alternating raised and depressed regions (e.g., a well which may retain solvents, reagents suitable for performing the methods of the present disclosure). In some cases, the surface of the substrate is divided into a number of separate sections and each individual section comprises a plurality of distinct locations and is configured to generate a different type of polymers (e.g., DNA, RNA, and organic polymers). The polymers may comprise any type of molecules having a number of monomers or subunits, e.g., nucleic acid molecules. The polymers can be single-stranded or double-stranded. In some cases, the polymers are selected from the group consisting of DNA, RNA, PNA, LNA, and a hybrid thereof.

The surface of the substrate may be modified to facilitate or aid in the generation or synthesis of polymers. For example, in cases where photolithographic techniques are employed, the substrate surface can be modified with photolabile protecting groups. Once the surface is illuminated through a photolithographic mask, reactive hydroxyl groups can be yielded in the illuminated regions and a monomer or a subunit of polymeric molecules can be attached thereon. By consecutively adding a monomer or a subunit to a preexisting strand, polymeric molecules are synthesized. In one example, a 3' activated deoxynucleoside, protected at the 5' hydroxyl with a photolabile group, is provided to the surface such that coupling occurs at sites that had been exposed to light. The protection at 5'-end of the deoxynucleoside is to prevent subsequent unwanted (photo) chemical reactions. The selective photodeprotection and coupling cycles can be reiterated until the desired set of probes is obtained. A variation of this process may use polymeric semiconductor photoresists, which are selectively patterned by photolithographic techniques, rather than using photolabile 5' protecting groups. In some cases, a photo-activated protective group is used as each monomer or subunit is added. Such photo-activated protective group is of itself sensitive to light and can be activated upon exposure to light.

As described above and elsewhere herein, the substrate surface may be divided into a number of spatially-separated sections, each of which may comprise a plurality of distinct locations. Depending on the applications, each section may be used to synthesize the same or a different type of polymers and the locations within different sections may or may not take the same shape, have the same area, and/or be arranged in the same pattern.

### Methods

Another aspect of the present disclosure provides a method for synthesizing an array of polymers on a substrate. The array of polymers may comprise at least 100, 500, 1,000, 2,000, 3,000, 4,000, 5,000, 6,000, 7,000, 8,000, 9,000, 10,000, 20,000, 30,000, 40,000, 50,000, 60,000, 70,000, 80,000, 90,000, 100,000, 150,000, 200,000, 250,000, 300,000, 350,000, 400,000, 450,000, 500,000, 600,000, 700,000, 800,000, 900,000, 1,000,000, 10,000,000, 20,000,000, 30,000,000, 40,000,000, 50,000,000, 60,000,000, 70,000,000, 80,000,000, 90,000,000, 100,000,000, 200,000,000, 300,000,000, 400,000,000, 500,000,000, 600,000,000, 700,000,000, 800,000,000, 900,000,000, 1,000,000,000, 2,000,000,000, 3,000,000,000, 4,000,000,000, 5,000,000,000, or more unique polymeric molecules. First, a substrate which may fit for the purposes of polymer synthesis may be provided. The substrate may comprise a plurality of distinct locations. Each of the locations may comprise at least one site that is capable of attaching a subunit of the polymers onto the substrate. Each location may be adjacent to at least one, two, three, four, five, or six other locations. Each location may or may not have the same size, shape, or area. In some cases, a certain percentage of the locations has the same or a different size, shape, and/or area, for example, greater than or equal to 10%, 20%, 30%, 40%, 50%, 60%, 70%, 80%, 85%, 90%, 95%, or 99% of the locations may have the same size, shape and/or area.

Next, a set of masks may be provided. Each mask of the set may be used for defining a different subset of distinct locations on the substrate. Each mask may comprise a plurality of openings, which define a pattern of active regions and inactive regions on the substrate. During polymer synthesis, subunits can only be added onto the locations within the active regions.

The openings may take various shapes, regular or irregular, such as square, rectangular, triangular, diamond, hexagonal, and circle. Each mask may have its own design of openings, which defines a distinct pattern of active and inactive regions on the substrate. The openings may or may not be aligned in a single direction. Each opening may cover an integer number of distinct locations on the substrate. For each mask, the openings may or may not be of the same shape. For each distinct location on the substrate, the set of masks collectively may define a unique string of synthetic steps or embedding (i.e., a sequence of subunits to be introduced onto the substrate) used to form the polymers in that location. Each mask may be used for at least one synthetic step for forming the polymers. In some cases, the set of masks are designed such that each pair of strings of synthetic steps (or embeddings) used to form the polymers at two adjacent locations differ from each other by a maximum number of synthetic steps, for example, by at most 500, 400, 300, 200, 100, 90, 80, 70, 60, 50, 45, 40, 35, 30, 25, 20, 18, 16, 14, 12, 10, 9, 8, 7, 6, 5, 4, 3, or 2 synthetic steps. In some cases, two strings of synthetic steps used to form polymers at two adjacent locations differ from each other by one and only one synthetic step. For example, each pair of embeddings used to synthesize neighboring polymers in two adjacent locations differs by one and only one exposure/non-exposure step.

For each mask, a certain percentage (e.g., 1%, 2%, 3%, 4%, 5%, 6%, 7%, 8%, 9%, 10%, 20%, 30%, 40%, 50%, 60%, 70%, 80%, 85%, 90%, 95% or more) or all of the openings may have the same length and/or width. In some cases, the length of the openings may be the same as the substrate. In some cases, the length of the openings may be less than that of the substrate such that one mask is only capable of masking a portion of the substrate. In cases where all of the openings have the same length, their widths may vary and one or more of the openings may or may not have the same width. For example, the width of the openings may be greater than or equal to about 1 nm, 10 nm, 50 nm, 100 nm, 250 nm, 500 nm, 750 nm, 1 µm, 2 µm, 3 µm, 4 µm, 5 µm, 6 µm, 7 µm, 8 µm, 9 µm, 10 µm, 20 µm, 40 µm, 60 µm, 80 µm, 100 µm, 200 µm, 300 µm, 400 µm, 500 µm, 600 µm, 700 µm, 800 µm, 900 µm, 1,000 µm, or more. In some cases, the width of the openings may be smaller than or equal to about 50 mm, 10 mm, 1,000 µm, 900 µm, 800 µm, 700 µm, 600 µm, 500 µm, 400 µm, 300 µm, 200 µm, 100 µm, 90 µm, 80 µm, 70 µm, 60 µm, 50 µm, 40 µm, 30 µm, 20 µm, 10 µm, 8 µm, 6 µm, 4 µm, 2 µm, 1 µm, or less. In some cases, the width of the openings may be between any of the two values described herein, for example, 12 µm.

The length of the openings may vary. In some cases, each of the openings has a length of greater than or equal to about 1 µm, 10 µm, 25 µm, 50 µm, 75 µm, 100 µm, 200 µm, 400 µm, 600 µm, 800 µm, 1,000 µm, 2,000 µm, 3,000 µm, 3,500 µm, 4,000 µm, 4,500 µm, 5,000 µm, 5,500 µm, 6,000 µm, 7,000 µm, 8,000 µm, 9,000 µm, 10,000 µm, or more. In some cases, the length of the opening may be smaller than or equal to about 50,000 µm, 25,000 µm, 10,000 µm, 8,000 µm, 7,000 µm, 6,500 µm, 6,000 µm, 5,500 µm, 5,000 µm, 4,500 µm, 4,000 µm, 3,000 µm, 2,000 µm, 1,000 µm, 800 µm, 600 µm, 400 µm, 200 µm, 100 µm or less. In some cases, the length of the openings may be between any of the two values described herein, for example, 4,900 µm.

To synthesize polymers having multiple segments, more than one set of masks may be provided and each set of masks may be used for synthesizing, for example, a specific segment of the polymers. For example, a first set of masks having openings of the same length but different widths may be used for forming a first segment of the polymers and a second set of masks having openings of the same width but different lengths may be used for forming a second segment of the polymers. The openings of the first set and the second set of masks may be aligned in a first direction and a second direction, respectively, and the first and the second directions can be orthogonal to each other. In some cases, the same set of masks for the first segment synthesis may be used to form the second segments of the polymers by rotating the masks 90 degrees. A third set of masks (or a separate mask) may be used in some situations for forming a third segment (e.g., a known sequence of polymers commonly shared by all the polymers) of the polymers, which mask(s) may be designed to subject all the locations to the polymer synthesis.

The mask can be formed of various materials, such as glass, silicon-based (e.g., silica nitrides, silica), polymeric, semiconductor, or metallic materials. In some cases, the mask comprises lithographic masks (or photomasks). Thickness of the mask may vary. In some cases, the mask may have a thickness of greater than or equal to 1 µm, 10 µm, 50 µm, 100 µm, 250 µm, 500 µm, 750 µm, 1 millimeter (mm), 2 mm, 3 mm, 4 mm, 5 mm, 6 mm, 7 mm, 8 mm, 9 mm, 10 mm, 15 mm, 20 mm, 25 mm, 30 mm, 35 mm, 40 mm, 45 mm, 50 mm, or more. In some cases, the mask may have a thickness of less than or equal to about 500 mm, 250 mm, 100 mm, 50 mm, 40 mm, 30 mm, 20 mm, 10 mm, 8 mm, 6 mm, 4 mm, 2 mm, 1 mm, 900 µm, 800 µm, 700 µm, 600 µm, 500 µm, 400 µm, 300 µm, 200 µm, 100 µm, or less. In some cases, thickness of the mask may be between any of the two values described herein, e.g., about 7.5 mm.

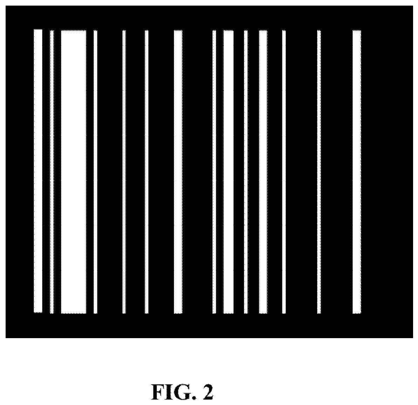

FIG. 2 illustrates an example mask of the present disclosure. As shown in FIG. 2, the openings (shown as white rectangular blocks) in the mask are used to synthesize the polymeric molecules (i.e., when the mask is placed above the substrate, locations under the openings are to be exposed and subjected to polymer synthesis). Each opening has a minimum width of 5 µm and can cover one or more locations on the substrate, depending upon, e.g., the dimension and area of each individual location. The mask is designed such that when it is aligned with respect to the substrate, selected locations on the substrate can be activated and subunits can be added thereon.

Next, a computer executable logic may be provided and used to (i) select a mask to overlay the substrate; and (ii) select one or more subunits to be introduced onto each location on the substrate using the mask. The computer executable logic that selects the mask the one or more subunits is configures to generate the polymer array(s). Each polymer synthesized on (and thus immobilized at) a distinct location on the substrate may have a unique sequence (or a string of subunits). Each polymer immobilized at a distinct location may differ from another immobilized at adjacent distinct locations in the sequence by a maximum number of subunits, for example, by at most 500, 400, 300, 200, 100, 90, 80, 70, 60, 50, 45, 40, 35, 30, 25, 20, 18, 16, 14, 12, 10, 9, 8, 7, 6, 5, 4, 3, or 2, including substitutions, insertions, deletions, and/or translocations ofsingle subunits. Subsequently, polymer synthesis may be performed using the selected masks and strings of subunits.

Various techniques can be used for synthesizing the polymers on the substrate, for example, chemical synthesis, electrochemical synthesis, or photoelctrochemical synthesis. In some cases, a light-directed synthesis is employed. A light source may be provided. The light source may be capable of performing the light-directed synthesis of the polymeric molecules on the substrate. The light source may provide various forms of radiations, such as visible light, ultraviolet light (UV), infrared (IR), extreme ultraviolet lithography (EUV), X-ray, electrons, and ions. The light source can provide a single wavelength, e.g. a laser, or a band of wavelengths. In some cases, the light beam provided by the light source may be in the range of ultraviolet to near ultraviolet wavelengths. A mask may be provided and positioned along an optical path between the light source and the substrate.

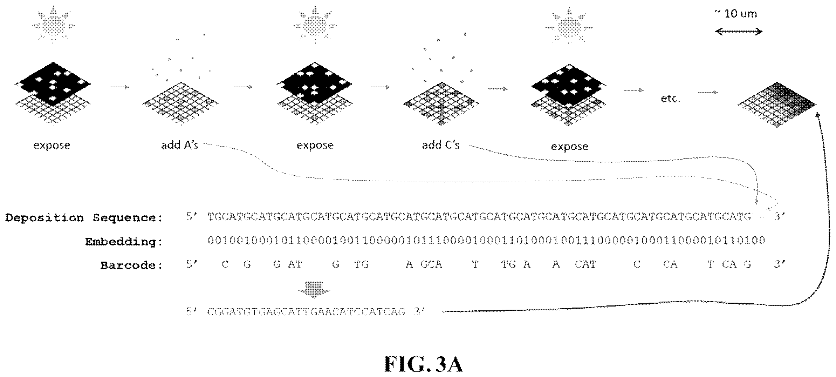

As described above and elsewhere herein, multiple synthetic steps may be included in the whole polymer synthetic process, and in some cases, for each individual step, there is one and only one mask that is selected and placed along the optical path between the substrate and the light source. In some cases, to synthesize polymeric molecules with pre-defined sequences of subunits, a set of masks can be used and the combination of the masks determines a set of strings of synthetic steps (a series of exposure and non-exposure steps) for all of the locations on the substrate. An example multi-step synthetic route of polymer arrays is shown in FIG. 3A.

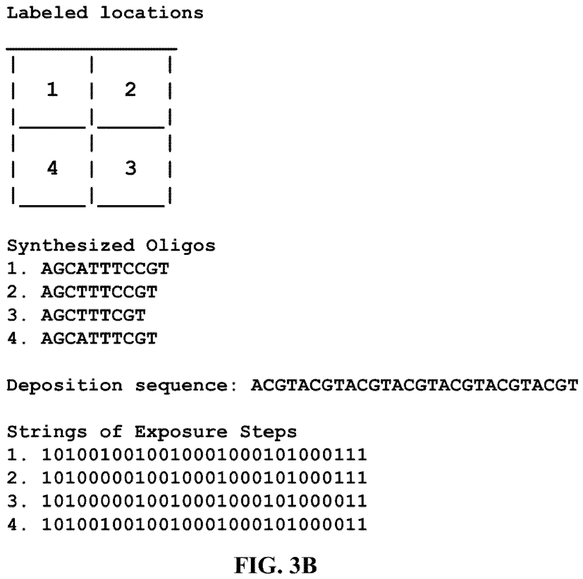

As provided herein, a computer system, as described in further detail below, may be utilized to generate a mask design file for producing physical masks for use in the synthetic reactions. The computer system may comprise a computer readable medium, which may comprise codes that, upon execution by one or more computer processors, implements a method for generating the mask design file. In some cases, a mask set may be designed such that all pairs of strings of synthetic steps for forming polymers in adjacent locations differ from each other by at most 500, 400, 300, 200, 100, 90, 80, 70, 60, 50, 45, 40, 35, 30, 25, 20, 18, 16, 14, 12, 10, 9, 8, 7, 6, 5, 4, 3, or 2 synthetic steps, for example, by one and only one synthetic step. This may greatly reduce the number of errors during synthesis. FIG. 3B illustrates such an example. As shown in FIG. 3B, each "string of exposure steps" is used to synthesize the corresponding synthesized oligo. In a given "string of exposure steps", a "1 " indicates that the corresponding location gets exposed to light during that step, and the corresponding subunit in the deposition sequence gets added to the synthesized oligo; a "0" indicates that the location does not get exposed and the subunit does not get added. For example, the first two 1's in the first "string of exposure steps" are at the first and third position, which correspond to an A and then a G in the deposition sequence, so the first two bases in the first oligo are "AG", etc. Each of the adjacent pairs of "strings of exposure steps" (e.g., locations land 2, 2 and 3, 3 and 4, and 4 and 1) differ from each other only by a single step. Although in the current example, "string of exposure steps" are represented by a string of "0"s and "1"s, it should be understood that various methods can be used to represent the "string of exposure steps".

Deposition sequences can be selected such that the number of synthetic steps (or cycles) can be minimized. In some cases, deposition sequences can be the repeated addition of certain short sequences (e.g., ACGT in the above example). The polymeric molecules can be synthesized with a sufficient long repetition of the deposition sequences until the molecules reach a pre-determined length.

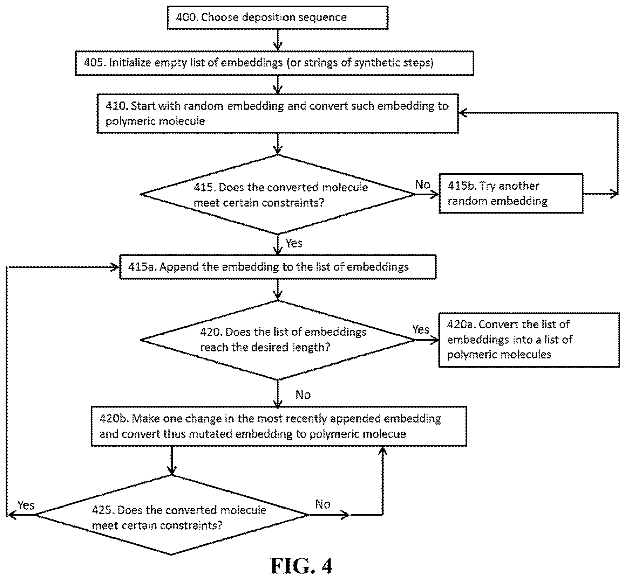

FIG. 4 illustrates an example work flow of generating a list of embeddings (or strings of synthetic steps). In a first operation 400, a deposition sequence to be used in the synthesis if chosen. Next, in a second operation 405, an empty list of embeddings (e.g., a series of exposure and non-exposure steps for a certain location) is utilized.

Following the creation of such list of embeddings, in a third operation 410, an embedding which represents mask steps for the first polymer is randomly picked. The selected embedding is then converted to the corresponding polymer. In some cases, the converted polymer is to be tested against several pre-defined constraints prior to the next operation 415. Examples of possible constraints may include, but not limited to, chain length; chemical, physical, thermal, electrical properties of polymers; biological properties such as GC and/or AT content in a particular range; ATG content in a certain range; nucleotide repeats; complexity; edit distance to reverse complement; edit distances to the other polymers implied by the embeddings in the list of embeddings; presence of forbidden sequences (e.g., sequences having a certain number of nucleotides in a row from the group consisting of G and C or A and T, sequences having a start codon, or sequence identical to the common, third segment); melting temperature; homopolymer runs beyond a certain range (or homopolymer limit); propensity for the formation of intramolecular secondary structures (e.g., hairpin structures); propensity for intermolecular annealing; exclusion of particular motifs (e.g., when using restriction enzymes); low similarity to genomic DNA; low similarity to mRNA sequence; and the like; and the combinations thereof. If the converted molecule meets the constraints 415a, then the selected embedding is added to the previously created empty list of the embeddings. Otherwise another random embedding has to be selected and tried 415b.

Following the step of 415a, once the list of embeddings reaches a desired length 420, it can be used for synthesizing polymeric molecules 420a such that the embeddings used to synthesize neighboring molecules in adjacent locations differ from each other by one and only one synthetic step (e.g., in light-directed polymer synthesis, all pairs of embeddings used for synthesizing polymers in adjacent locations differ by one and only 1 exposure/non-exposure step).

However, if the list of embeddings does not reach a per-determined length, then one change to the most recently appended embedding is made and the newly made embedding is converted to its corresponding polymeric molecule 420b. For example, if the embedding is represented by a string of "0"s and "1"s (e.g., "1010010010010001000101000111" as used in FIG. 3B), by "one change" we mean there is one and only one synthetic step such as exposure step (i.e., "1") or non-exposure step (i.e., "0") when light-directed synthesis is used that can be changed. Such operation 420b can be performed iteratively until the list of embeddings reaches a desired length. Each of converted molecules in step 420b may be optionally tested against certain constraints 425 and if it fails to meet one or more constraints, another random change is made 430. However, if the converted molecule passes the test, the embedding from which the molecule is made will be added to the list of embeddings and step 420-425 can be reiterated.

The synthesis process can be initiated by directing a light beam from the light source to the substrate in the mask pattern. The locations within the active regions may be exposed to the light beam and subjected to the light-directed synthesis of the polymeric molecules. The monomer or subunit of the polymers may be modified such that one terminus of the monomer (or subunit) is unreactive to further actions and each time there is one and only one monomer (or subunit) that can participate into the synthesis reactions.

A coordinate system can be provided to determine a position information (e.g., coordinate) for each of the locations on the substrate. With the coordinate, differences between sequences of subunits of polymers located at two distinct locations can be determined by, e.g., calculating a relative position (or a difference between the coordinates) of the two locations.

The polymer synthesis may be ceased till all of the masks have been selected and used. The synthetic steps of the synthesis reaction can be repeated until a certain percentage (e.g., greater than or equal to at least 10%, 20%, 30%, 40%, 50%, 55%, 60%, 65%, 70%, 75%, 80%, 85%, 90%, 91%, 92%, 93%, 94%, 95%, 96%, 97%, 98%, 99%, 99.5%, 99.9%, 99.99% or more) of the locations have at least one polymeric molecule attached thereon and/or the attached molecule meets certain pre-defined properties, such as chain length (e.g., a polymeric chain contains at least 10, 20, 30, 40, 50, 60, 70, 80, 90, 100 or more subunits), composition (e.g., a polymeric chain contains at least 20% of A, G, T, or C), GC contents (e.g., no more than 10%, 20%, 30%, 40%, 05 50%), edit distance between adjacent or neighboring molecules (e.g., neighboring molecules have an edit distance or a minimum distance of 1), or any of the constraints described above or elsewhere herein, or a combination thereof.

In some cases, the synthetic steps may be reiterated (e.g., for at least 5, 10, 25, 50, 75, 100, 200, 300, 400, 500, 600, 700, 800, 900, or more times) until the substrate has at least 10, 50, 100, 200, 400, 600, 800, 1,000, 2,000, 3,000, 4,000, 5,000, 6,000, 7,000, 8,000, 9,000, 10,000, 20,000, 30,000, 40,000, 50,000, 60,000, 70,000, 80,000, 90,000, 100,000, 200,000, 300,000, 400,000, 500,000, 1,000,000, 10,000,000, 20,000,000, 30,000,000, 40,000,000, 50,000,000, 60,000,000, 70,000,000, 80,000,000, 90,000,000, 100,000,000, 200,000,000, 300,000,000, 400,000,000, 500,000,000, 600,000,000, 700,000,000, 800,000,000, 900,000,000, 1,000,000,000, 2,000,000,000, 3,000,000,000, 4,000,000,000, 5,000,000,000, or more locations that have polymeric molecules synthesized thereon. In some cases, the number of locations that have molecules therein may be between any of the two values described herein, for example, 250,000.

### Computer systems

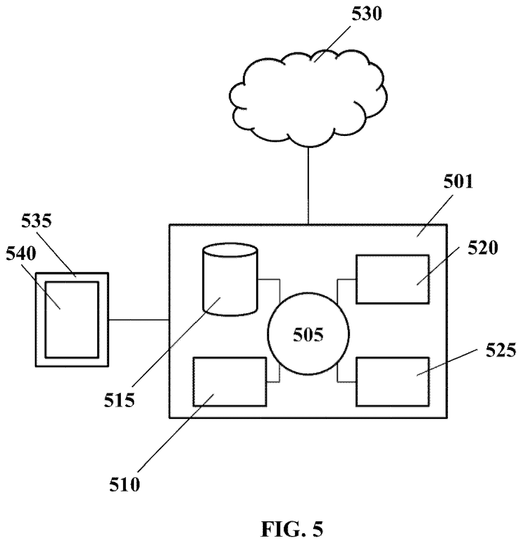

The present disclosure provides computer systems that are programmed or otherwise configured to implement methods provided herein, such as generating mask design file which defines a string of exposure steps for each individual location. Fig. 5 shows a computer system 501 that includes a central processing unit (CPU, also "processor" and "computer processor" herein) 505, which can be a single core or multi core processor, or a plurality of processors for parallel processing. The computer system 501 also includes memory or memory location 510 (e.g., random-access memory, read-only memory, flash memory), electronic storage unit 515 (e.g., hard disk), communication interface 520 (e.g., network adapter) for communicating with one or more other systems, and peripheral devices 525, such as cache, other memory, data storage and/or electronic display adapters. The memory 510, storage unit 515, interface 520 and peripheral devices 525 are in communication with the CPU 505 through a communication bus (solid lines), such as a motherboard. The storage unit 515 can be a data storage unit (or data repository) for storing data. The computer system 501 can be operatively coupled to a computer network ("network") 530 with the aid of the communication interface 520. The network 530 can be the Internet, an internet and/or extranet, or an intranet and/or extranet that is in communication with the Internet. The network 530 in some cases is a telecommunication and/or data network. The network 530 can include one or more computer servers, which can enable distributed computing, such as cloud computing. The network 530, in some cases with the aid of the computer system 501, can implement a peer-to-peer network, which may enable devices coupled to the computer system 501 to behave as a client or a server.

The CPU 505 can execute a sequence of machine-readable instructions, which can be embodied in a program or software. The instructions may be stored in a memory location, such as the memory 510. The instructions can be directed to the CPU 505, which can subsequently program or otherwise configure the CPU 505 to implement methods of the present disclosure. Examples of operations performed by the CPU 505 can include fetch, decode, execute, and writeback.

The CPU 505 can be part of a circuit, such as an integrated circuit. One or more other components of the system 501 can be included in the circuit. In some cases, the circuit is an application specific integrated circuit (ASIC).

The storage unit 515 can store files, such as drivers, libraries and saved programs. The storage unit 515 can store user data, e.g., user preferences and user programs. The computer system 501 in some cases can include one or more additional data storage units that are external to the computer system 501, such as located on a remote server that is in communication with the computer system 501 through an intranet or the Internet. The computer system 501 can communicate with one or more remote computer systems through the network 530.

Methods as described herein can be implemented by way of machine (e.g., computer processor) executable code stored on an electronic storage location of the computer system 501, such as, for example, on the memory 510 or electronic storage unit 515. The machine executable or machine readable code can be provided in the form of software. During use, the code can be executed by the processor 505. In some cases, the code can be retrieved from the storage unit 515 and stored on the memory 510 for ready access by the processor 505. In some situations, the electronic storage unit 515 can be precluded, and machine-executable instructions are stored on memory 510.

The code can be pre-compiled and configured for use with a machine having a processer adapted to execute the code, or can be compiled during runtime. The code can be supplied in a programming language that can be selected to enable the code to execute in a pre-compiled or as-compiled fashion.

The computer system 501 can be programmed or otherwise configured to regulate one or more parameters, such as the voltage applied across electrodes of a nano-gap electrode pair, temperature, flow rate of nucleic acid molecules, and time period for signal acquisition.

Aspects of the systems and methods provided herein, such as the computer system 501, can be embodied in programming. Various aspects of the technology may be thought of as "products" or "articles of manufacture" typically in the form of machine (or processor) executable code and/or associated data that is carried on or embodied in a type of machine readable medium. Machine-executable code can be stored on an electronic storage unit, such as memory (e.g., read-only memory, random-access memory, flash memory) or a hard disk. "Storage" type media can include any or all of the tangible memory of the computers, processors or the like, or associated modules thereof, such as various semiconductor memories, tape drives, disk drives and the like, which may provide non-transitory storage at any time for the software programming. All or portions of the software may at times be communicated through the Internet or various other telecommunication networks. Such communications, for example, may enable loading of the software from one computer or processor into another, for example, from a management server or host computer into the computer platform of an application server. Thus, another type of media that may bear the software elements includes optical, electrical and electromagnetic waves, such as used across physical interfaces between local devices, through wired and optical landline networks and over various air-links. The physical elements that carry such waves, such as wired or wireless links, optical links or the like, also may be considered as media bearing the software. As used herein, unless restricted to non-transitory, tangible "storage" media, terms such as computer or machine "readable medium" refer to any medium that participates in providing instructions to a processor for execution.

Hence, a machine readable medium, such as computer-executable code, may take many forms, including but not limited to, a tangible storage medium, a carrier wave medium or physical transmission medium. Non-volatile storage media include, for example, optical or magnetic disks, such as any of the storage devices in any computer(s) or the like, such as may be used to implement the databases, etc. shown in the drawings. Volatile storage media include dynamic memory, such as main memory of such a computer platform. Tangible transmission media include coaxial cables; copper wire and fiber optics, including the wires that comprise a bus within a computer system. Carrier-wave transmission media may take the form of electric or electromagnetic signals, or acoustic or light waves such as those generated during radio frequency (RF) and infrared (IR) data communications. Common forms of computer-readable media therefore include for example: a floppy disk, a flexible disk, hard disk, magnetic tape, any other magnetic medium, a CD-ROM, DVD or DVD-ROM, any other optical medium, punch cards paper tape, any other physical storage medium with patterns of holes, a RAM, a ROM, a PROM and EPROM, a FLASH-EPROM, any other memory chip or cartridge, a carrier wave transporting data or instructions, cables or links transporting such a carrier wave, or any other medium from which a computer may read programming code and/or data. Many of these forms of computer readable media may be involved in carrying one or more sequences of one or more instructions to a processor for execution.

The computer system 501 can include or be in communication with an electronic display 535 that comprises a user interface (UI) 540 for providing, for example, the progress of polymeric molecule synthesis. Examples of UI's include, without limitation, a graphical user interface (GUI) and web-based user interface.

Methods and systems of the present disclosure can be implemented by way of one or more algorithms. An algorithm can be implemented by way of software upon execution by the central processing unit 505.

### Applications

Methods and polymer arrays of the present disclosure may find useful in a wide variety of contexts, for example, multiplex assays or nucleic acid sequencing in biotechnology industries. Polymeric arrays produced by the methods described in the present disclosure may be used for tagging, tracking, identifying, and/or sequencing any sample or species, such as DNA or RNA molecules. For example, E. coli has a genome of approximately 4.6 Mb, which can be sequenced in one process. Sequencing larger segments of DNA or RNA, for example 50 kb or 100 kb, can accurately characterize some repeating sequences and larger structural changes, but can mischaracterize structural changes on the order of megabases. The methods and polymer arrays described herein can more accurately characterize repeating sequences, larger structural changes, and megabase-scale structural changes. The nucleic acid molecules sequenced can be entire genomes, for example E. coli genomes. The nucleic acid molecules sequenced can be very long strands of human DNA or chromosome.

A sample or species can be, for example, any substance used in sample processing, such as a reagent or an analyte. Exemplary samples may include whole cells, chromosomes, polynucleotides, organic molecules, proteins, polypeptides, carbohydrates, saccharides, sugars, lipids, enzymes, restriction enzymes, ligases, polymerases, barcodes, adaptors, small molecules, antibodies, fluorophores, deoxynucleotide triphosphate (dNTPs), dideoxynucleotide triphosphates (ddNTPs), buffers, acidic solutions, basic solutions, temperature-sensitive enzymes, pH-sensitive enzymes, light-sensitive enzymes, metals, metal ions, magnesium chloride, sodium chloride, manganese, aqueous buffer, mild buffer, ionic buffer, inhibitors, oils, salts, ions, detergents, ionic detergents, non-ionic detergents, oligonucleotides, nucleotides, DNA, RNA, peptide polynucleotides, complementary DNA (cDNA), double stranded DNA (dsDNA), single stranded DNA (ssDNA), plasmid DNA, cosmid DNA, chromosomal DNA, genomic DNA, viral DNA, bacterial DNA, mtDNA (mitochondrial DNA), mRNA, rRNA, tRNA, nRNA, siRNA, snRNA, snoRNA, scaRNA, microRNA, dsRNA, ribozyme, riboswitch and viral RNA, proteases, nucleases, protease inhibitors, nuclease inhibitors, chelating agents, reducing agents, oxidizing agents, probes, chromophores, dyes, organics, emulsifiers, surfactants, stabilizers, polymers, water, pharmaceuticals, radioactive molecules, preservatives, antibiotics, aptamers, and the like.

### Examples

### Example 1: Synthesis of polymeric molecules

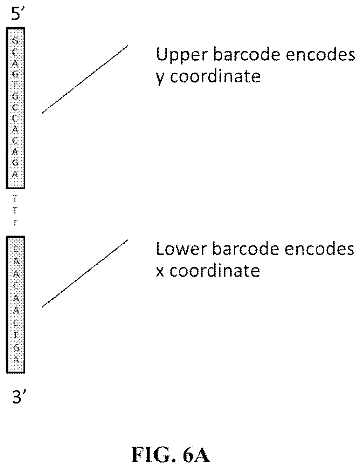

FIGs. 6A-6D illustrate an example procedure to synthesize polymeric molecules with three sets of masks. The polymeric molecules comprise a plurality of DNA barcodes, an example of which is shown in FIG. 6A. In FIG. 6A, the example DNA barcode comprises an upper barcode, a lower barcode separated by 3 T's.

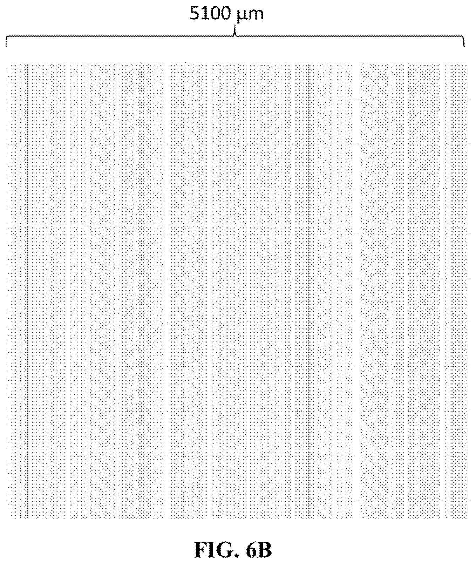

The DNA barcodes are synthesized with light-directed DNA synthesis method and the light exposure patterns are controlled by masks. The lower barcodes are synthesized first, and then the 3 T's are synthesized, followed by the synthesis of the upper barcodes. An example barcode for one step of lower barcode synthesis is illustrated in FIG. 6B, in which, the shaded rectangles represent transparent regions in the mask, defining the active regions of the locations on the substrate.

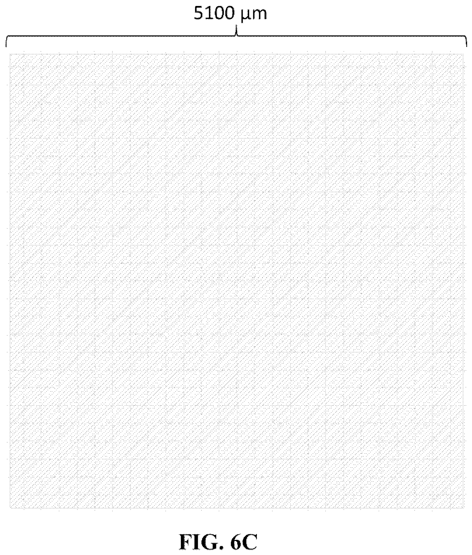

Once the synthesis of the lower barcodes is completed, the 3 T's are added to all of the synthesized barcodes during three identical synthetic steps. Thus, the mask used during these steps simply exposes all locations on the substrate (i.e., all areas of the lattice on which the locations are arranged). An example mask that can be used for such purpose is shown in FIG. 6C.

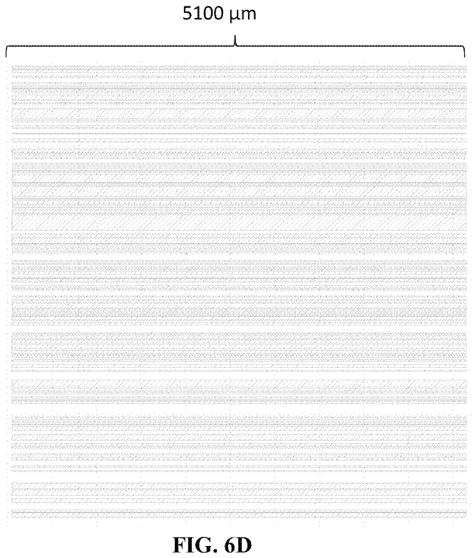

An example mask for one step of upper barcode synthesis is illustrated in FIG. 6D. Similar to FIG. 6B, the rectangles with diagonal lines therein represent the transparent regions in the mask, which then define the active regions on the substrate. In contrast to the mask utilized in synthetic steps for lower barcodes in which the rectangles are aligned vertically, the mask employed here comprise horizontally aligned rectangles.

### Example 2: Synthesis of polymeric molecules with a set of masks

When synthesizing a plurality of DNA barcodes on a substrate, a set of masks is used with a particular base added after each mask exposure. As described above, a deposition sequence may be provided for polymer synthesis. Each mask corresponds to exactly one subunit addition step. For a given location on the substrate, each mask will either expose or not expose that location such that the corresponding subunit in the deposition sequence is added or not getting added to the barcode to be synthesized at that location. The string of synthetic steps (or embeddings) of that location is encoded, for example, with 1's (exposures) and 0's (non-exposures).

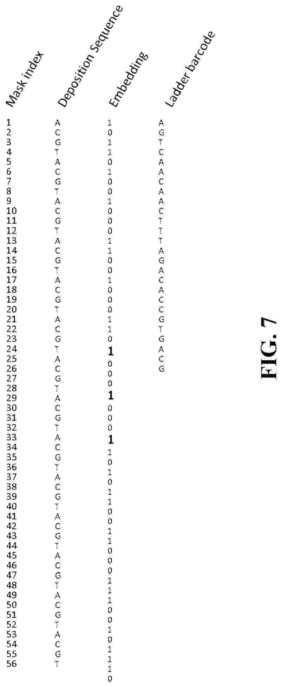

An example of an embedding and resulting barcodes is illustrated in FIG. 7. As the figure shows, the 3 T's is synthesized in the middle of the barcode, which separated the lower barcode form the upper barcode. The exposure steps corresponding to sequence of 3 T's are shown as 1's in bold.

### Example 3: Methods for generating a set of masks for lower DNA barcode synthesis

Generating a set of masks for lower ladder barcode synthesis consists of the following steps:
(1) choose a deposition sequence of nucleotides to be used during synthesis (e.g., ACGTACGT...);
(2) initialize an empty list of embeddings and determine an appropriate embedding length, *n*, for all the embeddings. The number *n* can also be the number of masks in the set of masks used for lower barcode synthesis; (3) start with a random embedding of length *n* (string of 0's and 1's). Convert this embedding to the corresponding lower barcode, and if the barcode meets the constraints (e.g., no string of consecutive T's), append the embedding to the list of embeddings; otherwise, keep trying random embeddings until one works; (4) create a candidate embedding by copying the most recently appended embedding with exactly one random change (a change of a 0 to a 1, or a 1 to a 0). Convert this candidate embedding to the corresponding lower barcode, and if the barcode meets the constraints (e.g., no string of consecutive T's, correct edit distance when compared to the other lower barcodes implied by the embeddings in the list), append the candidate embedding to the list of embeddings; (5) repeat step 4 until the list of embeddings reaches the intended length (e.g., if the resulting grid of barcodes is supposed to consist of a 5100 µm x 5100 µm grid of 1 µm squares, then we will need a list of 5100 embeddings). If Step 4 is repeated 100 consecutive times without another embedding appended to the list, then backtrack by removing the 10 most recently added embeddings from the list of embeddings and then continue to repeat step 4; (6) the list of embeddings is then converted into a set of *n* mask files (e.g., GDSII file format) by considering each of the digits in each of the embeddings. If the ith digit in the *j*th embedding is a 1, then a 5100 µm x 1 µm vertical rectangle is added to the ith mask file with x-coordinate *j*. If the ith digit in the *j*th embedding is a 0, then there is no need to add any rectangle. The resulting mask files will look like the illustration in FIG. 6B; and (7) the mask files are then converted into physical masks; all of the rectangular regions in a mask file correspond to transparent regions in the corresponding physical mask. The mask files can be converted into physical masks for photolithography by a company which produces photolithographic masks. Similar steps may be used to create masks for synthesizing upper barcodes of the polymeric molecules. In some cases, the same set of masks for lower barcode synthesis can be used for upper barcode synthesis by rotating the masks 90 degrees.

### Example 4: Method of generating embeddings for reducing risk of errors during polymer synthesis

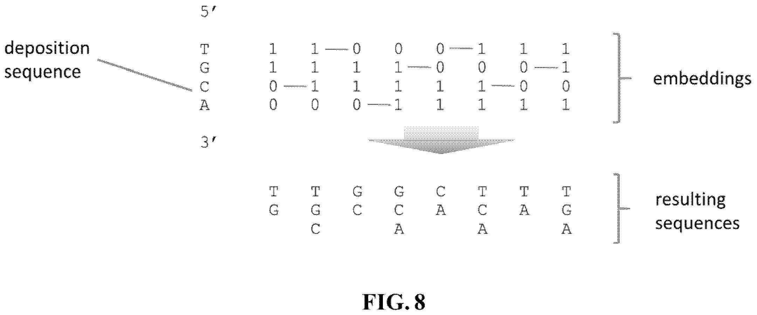

In some cases, misalignment of the masks relative to the substrate may occur and cause errors during synthesis, resulting in a mismatch between actual and desired polymeric sequences. In most instances, such misalignment only causes errors where neighboring embeddings differ from each other at certain synthetic steps. To reduce the risk of errors caused by the misalignment, it may be preferred to generate a set of embeddings with the overall number of differences between neighboring embeddings minimized, for example, the neighboring embeddings differ from each other by exactly one change. An example set of embeddings and resulting polymeric sequences are shown in FIG. 8.

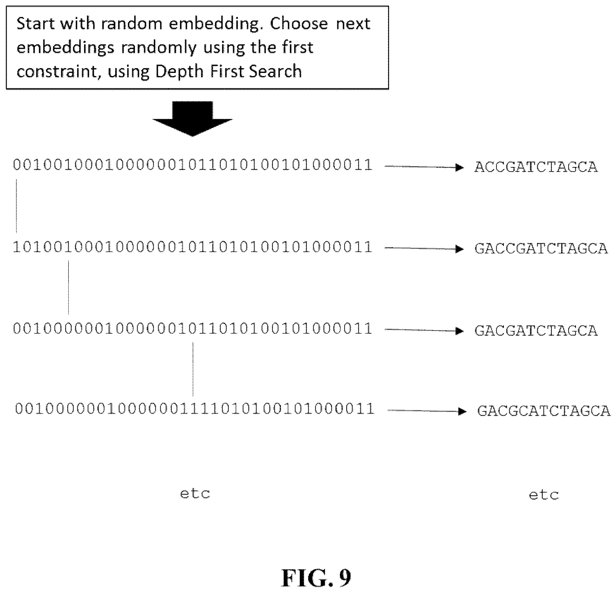

In some cases, it may be desired to have a plurality of polymers synthesized, which polymers have (1) roughly equal lengths, and (2) higher long-range minimum edit distance, The long-range minimum edit distance, as used herein, dictates for some given D, if two polymers are ≥D locations apart on the substrate, their edit distance must be ≥D. The synthesized polymers can be short or long. In cases where short sequences are needed, the synthetic route may comprise (i) generating embeddings of all possible lengths that meet the abovementioned two constraints, i.e., all resulting polymers have substantially the same length and higher long-range minimum edit distance, and (ii) using the shortest length that yields enough polymers for synthesis. An example method is illustrated in FIG. 9, which method starts with initializing an empty list of embedding and selecting a random embedding of length *n* (a string of 0's and 1's). Then, a candidate embedding is randomly chosen using the first constraint. After the number of embeddings included in the list reached a pre-determined value, the second constraint is applied and the embeddings failing to meet this constraint are removed from the list. Such generated embeddings can then be used for synthesizing polymer having a single segment.

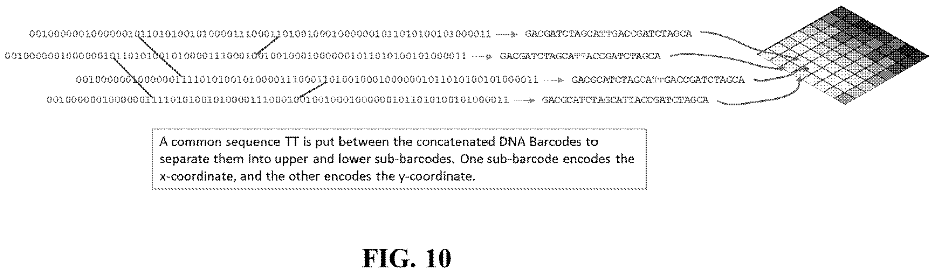

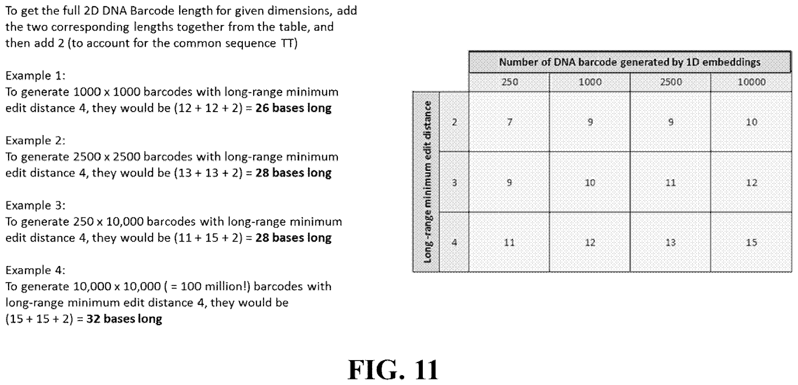

In some cases, two or more (e.g., 2, 3, 4, 5, 6, 7, 9, or 10) of the generated embeddings are concatenated to form a new set of embeddings that can be used for synthesizing polymers having multiple segments. Additionally or alternatively, a common known embedding with a much shorter length than (e.g., a string of 0's and 1's of length less than 2, 3, 4, 5, 6, 7, 8, 9, or 10) and distinguished from the concatenated embeddings may be inserted into neighboring concatenated embeddings to separate them, and each of the concatenated embeddings may correspond to a segment of the polymers. For example, as shown in FIG. 10, each embedding is generated by concatenating two previously generated embeddings (FIG. 9) and inserting a common string (i.e., 10001) between the concatenated embeddings. Each of the newly formed embeddings comprises three sections each corresponding to a single segment of the resulting polymers, e.g., an upper segment, a middle segment and a lower segment. The upper and the lower segments may encode the x- and y-coordinate respectively, and the middle segment is used for separating the upper and the lower segments. In some cases, the sets of embeddings prior to and after the concatenation are called 1D and 2D embeddings and the generated 1D and 2D embeddings can be separately used to design masks for synthesizing polymers that have a single and multiple segments respectively. FIG. 11 illustrates an example method for generating 2D DNA sequences using concatenated embeddings.

### Example 5: Methods for sequencing very long DNA using generated polymer arrays

A solution of DNA extract is prepared, comprising long pieces of template DNA molecules, approximately 4 Mb long. Primer binding sites are added to the template DNA molecules by transposon integration at an average spacing of 500 bp. The template DNA is stretched by molecular combing onto a substrate (such as glass slide) comprising a spatially-defined array of polymers, each coupled to a distinct location (or site) of the substrate. Each polymer of the array has an adaptor sequence complementary to the primer-binding site sequence of the template DNA molecule, a nucleic acid amplification primer sequence (e.g., PCR primer sequence), and a barcode sequence unique to the spot where the polymer is positioned. The polymers of the array hybridize to the primer binding sites previously integrated into the template DNA molecule. Extension reactions are conducted to generate multiple copies of regions of the template DNA molecules (or complements thereto), beginning at the 5' end with the PCR primer sequences of the polymers, next incorporating the barcode sequence, followed by incorporation of the adaptor sequence, and then extending to incorporate template nucleic acid sequence into the resulting extension product. Thus, array-bound extension products comprising barcode sequences and sequences complementary to regions of the template DNA molecules are produced. Extension products are assembled and sequenced. Alignment and assembly of the sequence reads is aided by the barcode information, and a complete 4 Mb template DNA sequence is produced.

While preferred embodiments of the present invention have been shown and described herein, it will be obvious to those skilled in the art that such embodiments are provided by way of example only. Numerous variations, changes, and substitutions will now occur to those skilled in the art. It should be understood that various alternatives to the embodiments of the invention described herein may be employed in practicing the disclosure. It is intended that the following claims define the scope of the invention and that methods and structures within the scope of these claims be covered thereby.

## Claims

1. An array comprising at least 1,000 different polynucleotides, each of the polynucleotides coupled to a distinct location on a surface in a two-dimensional pattern with rows and columns, wherein each of the polynucleotides differs from polynucleotides adjacent to it by at most 5 nucleotide subunits, wherein each of the polynucleotides comprises a first segment associated with a given row of the rows and a second segment associated with a given column of the columns of the distinct location.

2. The array of claim 1, wherein the array comprises at least 10,000 polynucleotides and each of the polynucleotides comprises at least 10 nucleotide subunits.

3. The array of claim 1 or 2, wherein each of the polynucleotides is adjacent to at least two other polynucleotides.

4. The array of any one of claims 1-3, wherein polynucleotides immobilized at two nonadjacent locations differ from each other by at least the same number of insertions, deletions, substitutions, and/or translocations of single nucleotide subunits as the number of locations between the two nonadjacent locations.

5. The array of any one of claims 1-4, wherein the two-dimensional pattern is with n rows and m columns, wherein n and m are at least 30.

6. The array of any one of claims 1-5, wherein the first segment is adjacent to the surface, wherein the second segment is distal to the surface, and wherein a third segment is between the first segment and the second segment and the third segment is the same for each of the polynucleotides, each of the segments comprising at least two nucleotide subunits.

7. The array of claim 5 or 6, wherein polynucleotides immobilized at adjacent locations in the same column/row have the same first/second segment and differ in the second/first segment by at most 5 nucleotide subunits.

8. The array of any one of claims 5-7, wherein polynucleotides immobilized at two nonadjacent locations in the same column/row have the same first/second segment and differ from each other in the second/first segment by at least the same number of insertions, deletions, substitutions, and/or translocations of single nucleotide subunits as the number of locations between the two nonadjacent locations.

9. The array of any one of claims 1-8, wherein each of the polynucleotides is located in an area of less than 100 µm².

10. A method for synthesizing an array of at least 1,000 polynucleotides each coupled to a distinct location on a substrate, comprising:
a. providing a substrate having a plurality of distinct locations in a two-dimensional pattern with rows and columns;
b. providing a set of masks, each mask of the set defining a different subset of the plurality of distinct locations on the substrate;
c. using a computer executable logic selecting a mask from the set of masks to overlay the substrate;
d. using the computer executable logic selecting one or more nucleotide subunits to be introduced onto the substrate at a defined subset of the plurality of distinct locations using the selected mask;
e. performing polynucleotide synthesis on the substrate at the defined subset of the plurality of distinct locations using the one or more nucleotide subunits; and
f. repeating steps (b)-(e) at least 10 times, thereby generating an array of at least 1,000 polynucleotides, each coupled to one of said plurality of distinct locations,
wherein each of the polynucleotides comprises a first segment associated with a given row of the rows and a second segment associated with a given column of the columns of the distinct location.

11. The method of claim 10, wherein each individual mask of the set comprises a plurality of openings aligned in a single direction which defines a pattern of active and inactive regions on the substrate, and wherein the one or more nucleotide subunits are only added to the active regions of the substrate during synthesis.

12. The method of claim 11, wherein each of the openings has a rectangular shape with a length of at least 500µm, and wherein at least 50% of the openings have differing widths.

13. The method of claim 10, wherein each of the polynucleotides is formed with a unique string of synthetic steps defined by the set of masks, and wherein each pair of the strings of synthetic steps used to form neighboring polynucleotides in adjacent locations differs from each other by at most 5 synthetic steps.

14. The method of any one of claims 10-13, wherein each of the polynucleotides of the array further comprises a third segment between the first segment and the second segment, each of the segments comprising at least two nucleotide subunits, wherein the third segment is the same for each of the polynucleotides, and wherein the same set of masks is used for forming both the first segment and the second segment of the polynucleotides, and a separate mask designed to expose all of the distinct locations on the substrate is provided for forming the third segment of the polynucleotides.

15. The method of any one of claims 10-14, wherein the array comprises at least 10,000 polynucleotides and each of the polynucleotides comprises at least 15 nucleotide subunits.

## Patentansprüche

1. Anordnung, die mindestens 1.000 verschiedene Polynukleotide umfasst, wobei jedes der Polynukleotide an eine bestimmte Stelle auf einer Oberfläche in einem zweidimensionalen Muster mit Zeilen und Spalten gekoppelt ist, wobei sich jedes der Polynukleotide von benachbarten Polynukleotiden um höchstens 5 Nukleotid-Untereinheiten unterscheidet, wobei jedes der Polynukleotide ein erstes Segment umfasst, das einer bestimmten Reihe der Reihen zugeordnet ist, und ein zweites Segment, das einer bestimmten Spalte der Spalten der bestimmten Stelle zugeordnet ist.

2. Anordnung nach Anspruch 1, wobei die Anordnung mindestens 10.000 Polynukleotide umfasst und jedes der Polynukleotide mindestens 10 Nukleotid-Untereinheiten umfasst.

3. Anordnung nach Anspruch 1 oder 2, wobei jedes der Polynukleotide mindestens zu zwei anderen Polynukleotiden benachbart angeordnet ist.

4. Anordnung nach einem der Ansprüche 1 bis 3, wobei sich Polynukleotide, die an zwei nicht benachbarten Stellen immobilisiert sind, durch mindestens die gleiche Anzahl von Insertionen, Deletionen, Substitutionen und/oder Translokationen einzelner Nukleotid-Untereinheiten wie die Anzahl von Stellen zwischen den zwei nicht benachbarten Stellen voneinander unterscheiden.

5. Anordnung nach einem der Ansprüche 1 bis 4, wobei das zweidimensionale Muster aus n Zeilen und m Spalten besteht, wobei n und m mindestens 30 sind.

6. Anordnung nach einem der Ansprüche 1 bis 5, wobei das erste Segment an die Oberfläche angrenzt, wobei das zweite Segment von der Oberfläche entfernt ist, und wobei ein drittes Segment zwischen dem ersten Segment und dem zweiten Segment liegt und das dritte Segment für jedes der Polynukleotide dasselbe ist, wobei jedes der Segmente mindestens zwei Nukleotid-Untereinheiten umfasst.

7. Anordnung nach Anspruch 5 oder 6, wobei Polynukleotide, die an benachbarten Stellen in derselben Spalte/Reihe immobilisiert sind, dasselbe erste/zweite Segment aufweisen und sich im zweiten/ersten Segment um höchstens 5 Nukleotid-Untereinheiten unterscheiden.

8. Anordnung nach einem der Ansprüche 5 bis 7, wobei Polynukleotide, die an zwei nicht benachbarten Stellen in derselben Spalte/Reihe immobilisiert sind, dasselbe erste/zweite Segment aufweisen und sich im zweiten/ersten Segment durch mindestens die gleiche Anzahl von Insertionen, Deletionen, Substitutionen und/oder Translokationen einzelner Nukleotid-Untereinheiten wie die Anzahl von Stellen zwischen den beiden nicht benachbarten Stellen voneinander unterscheiden.

9. Anordnung nach einem der Ansprüche 1 bis 8, wobei sich jedes der Polynukleotide in einer Fläche von weniger als 100 µm² befindet.

10. Verfahren zum Synthetisieren einer Anordnung von mindestens 1000 Polynukleotiden, die jeweils an eine bestimmte Stelle auf einem Substrat gekoppelt sind, wobei das Verfahren Folgendes umfasst:
a. Bereitstellen eines Substrats mit mehreren unterschiedlichen Stellen in einem zweidimensionalen Muster mit Zeilen und Spalten;
b. Bereitstellen eines Satzes von Masken, wobei jede Maske des Satzes eine andere Teilmenge der mehreren unterschiedlichen Stellen auf dem Substrat definiert;
c. Verwenden einer computerausführbaren Logik, die eine Maske aus dem Satz von Masken auswählt, um das Substrat zu überlagern;
d. Verwenden der computerausführbaren Logik zum Auswählen einer oder mehrerer Nukleotid-Untereinheiten, die unter Verwendung der ausgewählten Maske an einer definierten Untergruppe der mehreren unterschiedlichen Stellen auf das Substrat eingebracht werden sollen;
e. Durchführen einer Polynukleotidsynthese auf dem Substrat an der definierten Untermenge der Vielzahl von unterschiedlichen Stellen unter Verwendung der einen oder mehreren Nukleotid-Untereinheiten; und
f. mindestens zehnmaliges Wiederholen der Schritte (b) bis (e), wodurch eine Anordnung von mindestens 1000 Polynukleotiden erzeugt wird, die jeweils an eine der mehreren unterschiedlichen Stellen gekoppelt sind,
wobei jedes der Polynukleotide ein erstes Segment umfasst, das einer bestimmten Reihe der Reihen zugeordnet ist, und ein zweites Segment, das einer bestimmten Spalte der Spalten der bestimmten Stelle zugeordnet ist.

11. Verfahren nach Anspruch 10, wobei jede einzelne Maske des Satzes eine Vielzahl von Öffnungen umfasst, die in einer einzigen Richtung ausgerichtet sind, die ein Muster von aktiven und inaktiven Bereichen auf dem Substrat definiert, und wobei die eine oder mehreren Nukleotid-Untereinheiten nur zu den aktiven Regionen des Substrats während der Synthese hinzugefügt werden.

12. Verfahren nach Anspruch 11, wobei jede der Öffnungen eine rechteckige Form mit einer Länge von mindestens 500 µm aufweist und wobei mindestens 50 % der Öffnungen unterschiedliche Breiten aufweisen.

13. Verfahren nach Anspruch 10, wobei jedes der Polynukleotide mit einer eindeutigen Folge von Syntheseschritten gebildet wird, die durch den Satz von Masken definiert sind, und wobei sich jedes Paar der Folgen von Syntheseschritten, die zur Bildung benachbarter Polynukleotide an benachbarten Stellen verwendet werden, um höchstens 5 Syntheseschritte voneinander unterscheidet.

14. Verfahren nach einem der Ansprüche 10 bis 13, wobei jedes der Polynukleotide der Anordnung ferner ein drittes Segment zwischen dem ersten Segment und dem zweiten Segment umfasst, wobei jedes der Segmente mindestens zwei Nukleotid-Untereinheiten umfasst, wobei das dritte Segment für jedes der Polynukleotide dasselbe ist, und wobei der gleiche Satz von Masken zum Bilden sowohl des ersten Segments als auch des zweiten Segments der Polynukleotide verwendet wird, und wobei eine separate Maske, die dazu bestimmt ist, alle unterschiedlichen Stellen auf dem Substrat freizulegen, zum Bilden des dritten Segments der Polynukleotide vorgesehen ist.

15. Verfahren nach einem der Ansprüche 10 bis 14, wobei die Anordnung mindestens 10.000 Polynukleotide umfasst und jedes der Polynukleotide mindestens 15 Nukleotid-Untereinheiten umfasst.

## Revendications

1. Réseau comprenant au moins 1000 polynucléotides différents, chacun des polynucléotides étant couplé à un emplacement distinct sur une surface dans un motif bidimensionnel avec des rangées et des colonnes, chacun des polynucléotides se distinguant des polynucléotides qui lui sont adjacents d'au moins 5 sous-unités nucléotidiques, chacun des polynucléotides comprenant un premier segment associé à une rangée donnée des rangées et un deuxième segment associé à une colonne donnée des colonnes de l'emplacement distinct.

2. Réseau selon la revendication 1, le réseau comprenant au moins 10 000 polynucléotides et chacun des polynucléotides comprenant au moins 10 sous-unités nucléotidiques.

3. Réseau selon la revendication 1 ou 2, chacun des polynucléotides étant adjacent à au moins deux autres polynucléotides.

4. Réseau selon l'une quelconque des revendications 1 à 3, les polynucléotides immobilisés en deux emplacements non adjacents se distinguant l'un de l'autre d'au moins le même nombre d'insertions, de suppressions, de substitutions, et/ou de translocations de sous-unités nucléotidiques simples que le nombre d'emplacements entre les deux emplacements non adjacents.

5. Réseau selon l'une quelconque des revendications 1 à 4, le motif bidimensionnel étant constitué de n rangées et de m colonnes, n et m valant au moins 30.

6. Réseau selon l'une quelconque des revendications 1 à 5, le premier segment étant adjacent à la surface, le deuxième segment étant distal par rapport à la surface, et un troisième segment étant entre le premier segment et le deuxième segment et le troisième segment étant le même pour chacun des polynucléotides, chacun des segments comprenant au moins deux sous-unités nucléotidiques.

7. Réseau selon la revendication 5 ou 6, les polynucléotides immobilisés en des emplacements adjacents dans la même colonne/rangée possédant le même premier/deuxième segment et se distinguant dans le deuxième/premier segment d'au moins 5 sous-unités nucléotidiques.

8. Réseau selon l'une quelconque des revendications 5 à 7, les polynucléotides immobilisées en deux emplacements non adjacents dans la même colonne/rangée possédant le même premier/deuxième segment et se distinguant l'un de l'autre dans le deuxième/premier segment d'au moins le même nombre d'insertions, de suppressions, de substitutions, et/ou de translocations de sous-unités nucléotidiques simples que le nombre d'emplacements entre les deux emplacements non adjacents.

9. Réseau selon l'une quelconque des revendications 1 à 8, chacun des polynucléotides étant situé dans une aire de moins de 100 µm².

10. Procédé pour synthétiser un réseau d'au moins 1000 polynucléotides chacun couplé à un emplacement distinct sur un substrat, comprenant :
a. la fourniture d'un substrat possédant une pluralité d'emplacements distincts dans un motif bidimensionnel avec des rangées et des colonnes ;
b. la fourniture d'un ensemble de masques, chaque masque de l'ensemble définissant un sous-ensemble différent de la pluralité d'emplacements distincts sur le substrat ;
c. l'utilisation d'une logique exécutable par ordinateur sélectionnant un masque parmi l'ensemble de masques pour recouvrir le substrat ;
d. l'utilisation d'une logique exécutable par ordinateur sélectionnant au moins une sous-unité nucléotidique à introduire sur le substrat au niveau d'un sous-ensemble défini de la pluralité d'emplacements distincts à l'aide du masque sélectionné ;
e. l'exécution de la synthèse polynucléotidique sur le substrat au niveau du sous-ensemble défini de la pluralité d'emplacements distincts à l'aide de l'au moins une sous-unité ; et
f. la répétition des étapes (b) à (e) au moins 10 fois, pour ainsi générer un réseau d'au moins 1000 polynucléotides, chacun couplé à l'un de ladite pluralité d'emplacements distincts,
chacun des polynucléotides comprenant un premier segment associé à une rangée donnée des rangées et un deuxième segment associé à une colonne donnée des colonnes de l'emplacement distinct.

11. Procédé selon la revendication 10, chaque masque individuel de l'ensemble comprenant une pluralité d'ouvertures alignées dans une seule direction qui définit un motif de régions actives et inactives sur le substrat, et l'au moins une sous-unité nucléotidique étant seulement ajoutée aux régions actives du substrat durant la synthèse.

12. Procédé selon la revendication 11, chacune des ouvertures possédant une forme rectangulaire avec une longueur d'au moins 500 µm, et au moins 50 % des ouvertures possédants des largeurs différentes.

13. Procédé selon la revendication 10, chacun des polynucléotides étant formé par une unique chaîne d'étapes de synthèse définie par l'ensemble de masques, et chaque paire de chaînes d'étapes de synthèse utilisées pour former des polynucléotides voisins en des emplacements adjacents se distinguant l'une de l'autre de tout au plus 5 étapes de synthèse.

14. Procédé selon l'une quelconque des revendications 10 à 13, chacun des polynucléotides du réseau comprenant en outre un troisième segment entre le premier segment et le deuxième segment, chacun des segments comprenant au moins deux sous-unités nucléotidiques, le troisième segment étant le même pour chacun des polynucléotides, et le même ensemble de masques étant utilisé pour former à la fois le premier segment et le deuxième segment des polynucléotides, et un masque séparé conçu pour exposer toutes les emplacements distincts sur le substrat étant fourni pour former le troisième segment des polynucléotides.

15. Procédé selon l'une quelconque des revendications 10 à 14, le réseau comprenant au moins 10 000 polynucléotides et chacun des polynucléotides comprenant au moins 15 sous-unités nucléotidiques.


### SEQUENCE LISTING

<110> CENTRILLION TECHNOLOGY HOLDINGS CORPORATION
<120> METHODS FOR GENERATING POLYMER ARRAYS
<130> 38558-725.611
<140>
   <141>
<150> 62/254,589
   <151> 2015-11-12
<150> 62/204,937
   <151> 2015-08-13
<160> 21
<170> PatentIn version 3.5
<210> 1
   <211> 22
   <212> DNA
   <213> Artificial Sequence
<220>
   <221> source
   <223> /note="Description of Artificial Sequence: Synthetic oligonucleotide"
<400> 1
   agcgcttagc ctagagctct ag 22
<210> 2
   <211> 23
   <212> DNA
   <213> Artificial Sequence
<220>
   <221> source
   <223> /note="Description of Artificial Sequence: Synthetic oligonucleotide"
<400> 2
   gcgcttagct tagagctcta ttg 23
<210> 3
   <211> 13
   <212> DNA
   <213> Artificial Sequence
<220>
   <221> source
   <223> /note="Description of Artificial Sequence: Synthetic oligonucleotide"
<400> 3
   gcagtgccac aga 13
<210> 4
   <211> 10
   <212> DNA
   <213> Artificial Sequence
<220>
   <221> source
   <223> /note="Description of Artificial Sequence: Synthetic oligonucleotide"
<400> 4
   caacaactga 10
<210> 5
   <211> 76
   <212> DNA
   <213> Artificial Sequence
<220>
   <221> source
   <223> /note="Description of Artificial Sequence: Synthetic oligonucleotide"
<400> 5
   tgcatgcatg catgcatgca tgcatgcatg catgcatgca tgcatgcatg catgcatgca 60
   tgcatgcatg catgca 76
<210> 6
   <211> 27
   <212> DNA
   <213> Artificial Sequence
<220>
   <221> source
   <223> /note="Description of Artificial Sequence: Synthetic oligonucleotide"
<400> 6
   cggatgtgag cattgaacat ccatcag 27
<210> 7
   <211> 11
   <212> DNA
   <213> Artificial Sequence
<220>
   <221> source
   <223> /note="Description of Artificial Sequence: Synthetic oligonucleotide"
<400> 7
   agcatttccg t 11
<210> 8
   <211> 10
   <212> DNA
   <213> Artificial Sequence
<220>
   <221> source
   <223> /note="Description of Artificial Sequence: Synthetic oligonucleotide"
<400> 8
   agctttccgt 10
<210> 9
   <211> 10
   <212> DNA
   <213> Artificial Sequence
<220>
   <221> source
   <223> /note="Description of Artificial Sequence: Synthetic oligonucleotide"
<400> 9
   agcatttcgt 10
<210> 10
   <211> 28
   <212> DNA
   <213> Artificial Sequence
<220>
   <221> source
   <223> /note="Description of Artificial Sequence: Synthetic oligonucleotide"
<400> 10
   acgtacgtac gtacgtacgt acgtacgt 28
<210> 11
   <211> 26
   <212> DNA
   <213> Artificial Sequence
<220>
   <221> source
   <223> /note="Description of Artificial Sequence: Synthetic oligonucleotide"
<400> 11
   gcagtgccac agatttcaac aactga 26
<210> 12
   <211> 56
   <212> DNA
   <213> Artificial Sequence
<220>
   <221> source
   <223> /note="Description of Artificial Sequence: Synthetic oligonucleotide"
<400> 12
   acgtacgtac gtacgtacgt acgtacgtac gtacgtacgt acgtacgtac gtacgt 56
<210> 13
   <211> 26
   <212> DNA
   <213> Artificial Sequence
<220>
   <221> source
   <223> /note="Description of Artificial Sequence: Synthetic oligonucleotide"
<400> 13
   agtcaacaac tttagacacc gtgacg 26
<210> 14
   <211> 12
   <212> DNA
   <213> Artificial Sequence
<220>
   <221> source
   <223> /note="Description of Artificial Sequence: Synthetic oligonucleotide"
<400> 14
   accgatctag ca 12
<210> 15
   <211> 13
   <212> DNA
   <213> Artificial Sequence
<220>
   <221> source
   <223> /note="Description of Artificial Sequence: Synthetic oligonucleotide"
<400> 15
   gaccgatcta gca 13
<210> 16
   <211> 12
   <212> DNA
   <213> Artificial Sequence
<220>
   <221> source
   <223> /note="Description of Artificial Sequence: Synthetic oligonucleotide"
<400> 16
   gacgatctag ca 12
<210> 17
   <211> 13
   <212> DNA
   <213> Artificial Sequence
<220>
   <221> source
   <223> /note="Description of Artificial Sequence: Synthetic oligonucleotide"
<400> 17
   gacgcatcta gca 13
<210> 18
   <211> 27
   <212> DNA
   <213> Artificial Sequence
<220>
   <221> source
   <223> /note="Description of Artificial Sequence: Synthetic oligonucleotide"
<400> 18
   gacgatctag cattgaccga tctagca 27
<210> 19
   <211> 26
   <212> DNA
   <213> Artificial Sequence
<220>
   <221> source
   <223> /note="Description of Artificial Sequence: Synthetic oligonucleotide"
<400> 19
   gacgatctag cattaccgat ctagca 26
<210> 20
   <211> 28
   <212> DNA
   <213> Artificial Sequence
<220>
   <221> source
   <223> /note="Description of Artificial Sequence: Synthetic oligonucleotide"
<400> 20
   gacgcatcta gcattgaccg atctagca 28
<210> 21
   <211> 27
   <212> DNA
   <213> Artificial Sequence
<220>
   <221> source
   <223> /note="Description of Artificial Sequence: Synthetic oligonucleotide"
<400> 21
   gacgcatcta gcattaccga tctagca 27